(19)

Europäisches
Patentamt
European
Patent Office
Office européen
des brevets

(11) **EP 2 494 959 B1**

(12) **EUROPEAN PATENT SPECIFICATION**

(45) Date of publication and mention
of the grant of the patent:
**19.11.2014 Bulletin 2014/47**

(51) Int Cl.:
*A61K 9/12* *(2006.01)*    *A61K 9/107* *(2006.01)*
*A61K 8/04* *(2006.01)*    *A61K 47/12* *(2006.01)*
*A61K 47/14* *(2006.01)*

(21) Application number: **12162257.5**

(22) Date of filing: **05.07.2007**

(54) **Dicarboxylic acid foamable vehicle and pharmaceutical compositions thereof**

Dicarbonsäureschäumbare Trägersubstanz und pharmazeutische Zusammensetzungen daraus

Véhicule moussant à base d'acide dicarboxylique et ses compositions pharmaceutiques

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **05.07.2006 US 818634 P**

(43) Date of publication of application:
**05.09.2012 Bulletin 2012/36**

(62) Document number(s) of the earlier application(s) in
accordance with Art. 76 EPC:
**07858941.3 / 2 051 697**

(73) Proprietor: **Foamix Ltd.
74140 Ness Ziona (IL)**

(72) Inventors:
• **Berman, Tal
75278 Rishon Lezion (IL)**

• **Ziv, Enbal
Gedera 74140 (IL)**
• **Schuz, David
Gimzu 73130 (IL)**
• **Tamarkin, Dov
71908 Maccabim (IL)**
• **Friedman, Doron
Karmei-Yosef 99797 (IL)**

(74) Representative: **Adamson Jones
BioCity Nottingham
Pennyfoot Street
Nottingham
Nottinghamshire NG1 1GF (GB)**

(56) References cited:
**EP-A- 0 979 654      WO-A-01/82880
WO-A-03/055445      WO-A-2007/085899
US-A1- 2004 241 099      US-A1- 2005 031 547
US-A1- 2005 205 086**

EP 2 494 959 B1

## Description

### BACKGROUND OF THE INVENTION

This invention relates to foamable pharmaceutical and cosmetic compositions.

External topical administration is an important route for the administration of drugs in disease treatment. Many groups of drugs, including, for example, antibiotic, anti-fungal, anti-inflammatory, anesthetic, analgesic, anti-allergic, corticosteroid, retinoid and anti-proliferative medications are preferably administered in hydrophobic media, namely ointment. However, ointments often form an impermeable barrier, so that metabolic products and excreta from the wounds to which they are applied are not easily removed or drained away. Furthermore, it is difficult for the active drug dissolved in the carrier to pass through the white petrolatum barrier layer into the wound tissue, so the efficacy of the drug is reduced. In addition, ointments and creams often do not create an environment for promoting respiration of the wound tissue and it is not favorable to the normal respiration of the skin. An additional disadvantage of petroleum jelly-based products relates to the greasy feeling left following their topical application onto the skin, mucosal membranes and wounds.

Foams are considered a more convenient vehicle for topical delivery of active agents. There are several types of topical foams, including aqueous foams, such as commonly available shaving foams; hydroalcoholic foams, emulsion-based foams, comprising oil and water components, and oleaginous foams, which consist of high oil content. In skin therapy, oil containing foams are preferred, since oil contributes to skin protection and moisturization, which improve the therapeutic effect of the formulation.

Dicarboxylic acids are known to possess therapeutic properties. Dicarboxylic acids, and their mercapto, ester and salt derivatives have been used in the treatment of a variety of skin disorders and/or conditions.

Azelaic acid (AZA) is a naturally occurring nine carbon straight chain molecule with two terminal carboxyl groups. AZA is an anti-keratinizing agent, displaying antiproliferative effects on keratinocytes and modulating the early and terminal phases of epidermal differentiation. AZA is a competitive inhibitor of the reduction of testosterone to dihydrotestosterone, and as such is supposed to reduce the production of sebum in the sebaceous gland. Furthermore, recent investigations have demonstrated that AZA and sebacic acid also have anti-bacterial and anti-fungal properties. Structure-activity relationship studies have revealed that these effects are retained when the dicarboxylic acid has a backbone of about 6 to about 14 carbons.

Dicarboxylic acid esters are also known to contribute to the skin penetration of an active agent. Enhancing effects on skin penetration of methyl nicotinate have been observed with dibutyl adipate and dioctyl adipate. Diisopropyl sebacate also markedly enhances the skin penetration of the erythromycin. The skin penetration enhancing properties of mono- or di-esters of dicarboxylic acid, including dibutyl adipate, diethyl sebacate, diisopropyl dimerate, diisopropyl adipate, diisopropyl sebacate and dioctyl succinate have been recognized.

There remains an unmet need for improved, easy to use, stable oil-containing foam formulations, containing oils, which effectively deliver and/or deposit various benefit agents into and onto the skin and/or other target sites and are relatively non-irritating and thus suitable for use by people having sensitive skin and eyes.

### SUMMARY

The present invention relates to aqueous and non aqueous stable compositions comprising azelaic acid.

There is provided a pharmaceutical or cosmetic composition as defined in Claim 1.There is also provided a foamable composition as described above wherein the composition is contained in a pressurized container and further comprises a liquefied hydrocarbon gas propellant at a concentration of about 3% to about 25% by weight of the total composition, is substantially flowable and provides a foam upon release and wherein the azelaic acid, stabilizer and solvent are selected to generate a breakable foam of good to excellent quality.

The compositions described above may be in a non foam state.

There is also provided the use of the compositions in the preparation of a medicament for use in the treatment of dermatological disorders.

### DETAILED DESCRIPTION

According to one or more embodiments of the present invention, the composition includes:

    a. a benefit agent which is azelaic acid;
    b. a surface-active agent;
    c. about 0.01 % to about 5% by weight of at least one polymeric agent selected from the group consisting of a bioadhesive agent, a gelling agent, a film forming agent and a phase change agent; and

d. water.

The present invention further relates to a foamable composition including:

a. a benefit agent which is azelaic acid;
b. a surface-active agent;
c. about 0.01 % to about 5% by weight of at least one polymeric agent selected from the group consisting of a bioadhesive agent, a gelling agent, a film forming agent and a phase change agent;
d. water; and
e. liquefied hydrocarbon gas propellant at a concentration of about 3% to about 25% by weight of the total composition.

In one or more embodiments there is provided a foamable composition which produces a foam upon release and wherein the azelaic acid, stabilizer and solvent are selected to generate a breakable foam of good to excellent quality.

In one or more embodiments there is provided a composition wherein the azelaic acid, stabilizer and solvent are selected to generate an emulsion that is substantially resistant to phase reversal.

In one or more embodiments there is provided a composition wherein, the azelaic acid, stabilizer and solvent are selected to generate a single phase.

In one or more embodiments there is provided a composition wherein, the azelaic acid, stabilizer and solvent are selected to generate a substantially uniform suspension of benefit agent crystals.

In one or more embodiments there is provided a composition, wherein the breakable foam comprises micro or nano particles, crystals or bodies.

In one or more embodiments there is provided a composition, which is substantially resistant to one or more Freeze-Thaw cycles (FTC).

In one or more embodiments there is provided a composition wherein the surface-active agent is a solid, a liquid or a mixture thereof.

In one or more embodiments there is provided a composition wherein the surface active agent is selected from the group consisting of a polysorbate, polyoxyethylene (20) sorbitan monostearate, polyoxyethylene (20) sorbitan monooleate, a polyoxyethylene fatty acid ester, Myrj 45, Myrj 49, Myrj 52 and Myrj 59; a polyoxyethylene alkyl ether, polyoxyethylene cetyl ether, polyoxyethylene palmityl ether, polyethylene oxide hexadecyl ether, polyethylene glycol cetyl ether, brij 38, brij 52, brij 56 and brij W1, a sucrose ester, a partial ester of sorbitol, sorbitan monolaurate, a monoglyceride, a diglyceride, isoceteth-20, steareth 2, glyceryl monostearate/PEG 100 stearate, glyceryl stearate, steareth-21, PEG 40 stearate, polysorbate 60, polysorbate 80, sorbitan stearate, laureth 4, sorbitan monooleate, ceteareth 20, steareth 20, ceteth 20, macrogol cetostearyl ether, ceteth 2, PEG-30 dipolyhydroxystearate, sucrose distearate, polyoxyethylene (100) stearate, PEG 100 stearate, cetomacrogol ether, cetearyl glucoside, steareth-2, polysorbate 20; Montanov 68 (cetearyl alcohol (and) cetearyl glucoside), Simusol 165 (glyceryl stearate and PEG-100 stearate), methyl glucose sequistearate, PEG 30 dipolyhydroxystearate, sucrose stearic acid esters, sorbitan laureth, and mixtures thereof.

In one or more embodiments there is provided a composition wherein the surface active agent comprises at least one ester based surfactant or at least one ether based surfactant.

In one or more embodiments there is provided a composition wherein the surface active agent is reduced about in proportion to the increase in dicarboxylic ester.

In one or more embodiments there is provided a composition wherein the stabilizer is not a polymeric agent.

In one or more embodiments there is provided a composition wherein the surface active agent comprises a non-ionic surfactant that does not contain a polyoxyethylene (POE) moiety.

In one or more embodiments there is provided a composition wherein the surface active agent is selected from the group consisting of a non-ethoxylated sorbitan ester, a glycerol fatty acid ester, a sucrose ester and an alkyl polyglycoside or is selected from the group consisting of sorbitan monopalmitate, sorbitan monostearate, sorbitan tristearate, sorbitan monooleate, sorbitan trioleate, sorbitan monolaurate, sorbitan sesquioleate, glycerol monostearate, glycerol monooleate, sucrose stearate, sucrose distearate, sucrose palmitate sucrose laurate and lauryl diglucoside.

In one or more embodiments there is provided a composition wherein the polymeric agent is selected from the group consisting of carbopol 934, pemulen TR2, klucel EF, xanthan gum, methocel A4M, and carboxy methyl cellulose or selected from the group consisting of locust bean gum, sodium alginate, sodium caseinate, egg albumin, gelatin agar, carrageenin gum, sodium alginate, xanthan gum, quince seed extract, tragacanth gum, guar gum, cationic guars, hydroxypropyl guar gum, starch, an amine-bearing polymer, chitosan, alginic acid, hyaluronic acid, a chemically modified starch, a carboxyvinyl polymer, polyvinylpyrrolidone, polyvinyl alcohol, a polyacrylic acid polymer, a polymethacrylic acid polymer, polyvinyl acetate, a polyvinyl chloride polymer, a polyvinylidene chloride polymer, methylcellulose, hydroxypropyl cellulose, hydroxypropyl methylcellulose, hydroxyethyl cellulose, hydroxy propylmethyl cellulose, methylhydroxyethylcellulose, methylhydroxypropylcellulose, hydroxyethylcarboxymethylcellulose, carboxymethyl cellulose, carboxymethylhydroxyethylcellulose, a cationic cellulose aluminum starch octenylsuccinate (ASOS), PEG 1000, PEG 4000,

3

PEG 6000 and PEG 8000.

In one or more embodiments there is provided a composition wherein the polymeric agent is a derivatized polymer.

In one or more embodiments there is provided a composition wherein the derivatized polymer is a polymeric emulsifier.

In one or more embodiments there is provided a composition wherein, further comprising an additional active agent.

In one or more embodiments there is provided a composition wherein further containing a foam adjuvant selected from the group consisting of a fatty alcohol having 15 or more carbons in their carbon chain; a fatty acid having 16 or more carbons in their carbon chain; fatty alcohols, derived from beeswax and including a mixture of alcohols, a majority of which has at least 20 carbon atoms in their carbon chain; a fatty alcohol having at least one double bond; a fatty acid having at least one double bond; a branched fatty alcohol; a branched fatty acid and a fatty acid substituted with a hydroxyl group.

In one or more embodiments there is provided a composition wherein further containing at least one organic carrier selected from the group consisting of a hydrophobic organic carrier, an emollient and mixtures thereof, at a concentration of about 2% to about 50% by weight.

In one or more embodiments there is provided a composition wherein the azelaic acid is in a concentration between about 0.1 % and about 60%.

In one or more embodiments there is provided a composition wherein the concentration of azelaic acid is between 5% and 25%.

In one or more embodiments there is provided a composition wherein the pH of the composition is below the first pKa of the azelaic acid.

In one or more embodiments there is provided a composition wherein the pH of the composition is between the first and second pKa of the azelaic acid.

In one or more embodiments there is provided a composition wherein the pH of the composition is above the second pKa of the azelaic acid.

In one or more embodiments there is provided a composition wherein the pH of the composition is below 5.3.

In one or more embodiments there is provided a composition wherein the pH of the composition is between about 4.5 and about 5.3.

In one or more embodiments there is provided a composition wherein the azelaic acid, stabilizer and solvent are selected to generate an emulsion that can produce a substantially strong and closed packed barrier between the oil and the water phases whilst maintaining a fluid constitution

In one or more embodiments there is provided a composition further comprising an additional component selected from the group consisting of a modulating agent, a polar solvent, an anti perspirant, an anti-static agent, a buffering agent, a bulking agent, a chelating agent, a colorant, a conditioner, a deodorant, a diluent, a dye, an emollient, fragrance, a humectant, an occlusive agent, a penetration enhancer, a perfuming agent, a permeation enhancer, a pH-adjusting agent, a preservative, a skin penetration enhancer, a sunscreen, a sun blocking agent, a sunless tanning agent, and a vitamin.

In one or more embodiments there is provided a therapeutic composition comprising therapeutically effective amount of an active agent; and a beneficially or therapeutically effective concentration of azelaic acid wherein the active agent is selected from the group consisting of active herbal extracts, acaricides, age spot and keratose removing agents, allergen, analgesics, local anesthetics, antiacne agents, antiallergic agents, antiaging agents, antibacterials, antibiotics, antiburn agents, anticancer agents, antidandruff agents, antidepressants, antidermatitis agents, antiedemics, antihistamines, antihelminths, antihyperkeratolyte agents, antiinflammatory agents, antiirritants, antilipemics, antimicrobials, antimycotics, antiproliferative agents, antioxidants, anti-wrinkle agents, antipruritics, antipsoriatic agents, antirosacea agents antiseborrheic agents, antiseptic, antiswelling agents, antiviral agents, antiyeast agents, astringents, topical cardiovascular agents, chemotherapeutic agents, corticosteroids, dicarboxylic acids, disinfectants, fungicides, hair growth regulators, hormones, hydroxy acids, immunosuppressants, immunoregulating agents, insecticides, insect repellents, keratolytic agents, lactams, metals, metal oxides, mitocides, neuropeptides, non-steroidal anti-inflammatory agents, oxidizing agents, pediculicides, photodynamic therapy agents, retinoids, sanatives, scabicides, self tanning agents, skin whitening agents, vasoconstrictors, vasodilators, vitamins, vitamin D derivatives, wound healing agents and wart removers.

In one or more embodiments there is provided a foamable therapeutic composition wherein a dicarboxylic acid ester is present in the composition in an amount sufficient to solubilize the active agent.

In one or more embodiments there is provided a foamable therapeutic composition wherein the active agent is a steroid.

In one or more embodiments there is provided a foamable therapeutic composition wherein the steroid is selected from the group consisting of bydrocortisone, hydroxyltriamcinolone, alpha-methyl dexamethasone, dexamethasone-phosphate, beclomethsone dipropionate, clobetasol valemate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylester, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylpropionate, hydrocortmate, mepreddisone, paramethasone, prednisolone, prednisone, beclomethasone dipropionate, triamcinolone.

In one or more embodiments there is provided a foamable therapeutic composition wherein the active agent is an immunomodulator.

In one or more embodiments there is provided a foamable therapeutic composition, wherein the immunomodulator is selected from the group consisting of a cyclic peptides, cyclosporine, tacrolimus, tresperimus, pimecrolimus, sirolimus, verolimus, laflunimus, laquinimod and imiquimod.

In one or more embodiments there is provided a foamable therapeutic composition, wherein the dicarboxylic acid ester is present in the composition in an amount sufficient to solubilize the immunomodulator.

In one or more embodiments there is provided a foamable therapeutic composition, wherein the dicarboxylic acid ester is diisopropyl adipate.

In one or more embodiments there is provided a composition wherein the surface active agent comprises a non-ionic surfactant that does not contain a polyoxyethylene (POE) moiety.

In one or more embodiments there is provided a composition wherein the surface active agent is selected from the group consisting of a non-ethoxylated sorbitan ester, a glycerol fatty acid ester, a sucrose ester and an alkyl polyglycoside or is selected from the group consisting of sorbitan monopalmitate, sorbitan monostearate, sorbitan tristearate, sorbitan monooleate, sorbitan trioleate, sorbitan monolaurate, sorbitan sesquioleate, glycerol monostearate, glycerol monooleate, sucrose stearate, sucrose distearate, sucrose palmitate sucrose laurate and lauryl diglucoside.

In one or more embodiments, the composition of the invention may be used in a method of treating a disorder of a mammalian subject, comprising:

administering a foamable therapeutic composition to a target site, the composition comprising a therapeutically effective amount of an active agent; and a beneficially or therapeutically effective concentration of azelaic acid; wherein the target site is selected from the group consisting of the skin, a body cavity, a mucosal surface, the nose, the mouth, the eye, the ear canal, the respiratory system, the vagina and the rectum.

The disorder may be selected from the group consisting of dermatological pain, dermatological inflammation, acne, acne vulgaris, inflammatory acne, non-inflammatory acne, acne fulminans, nodular papulopustular acne, acne conglobata, dermatitis, bacterial skin infections, fungal skin infections, viral skin infections, parasitic skin infections, skin neoplasia, skin neoplasms, pruritis, cellulitis, acute lymphangitis, lymphadenitis, erysipelas, cutaneous abscesses, necrotizing subcutaneous infections, scalded skin syndrome, folliculitis, furuncles, hidradenitis suppurativa, carbuncles, paronychial infections, rashes, erythrasma, impetigo, ecthyma, yeast skin infections, warts, molluscum contagiosum, trauma or injury to the skin, post-operative or post-surgical skin conditions, scabies, pediculosis, creeping eruption, eczemas, psoriasis, pityriasis rosea, lichen planus, pityriasis rubra pilaris, edematous, erythema multiforme, erythema nodosum, granuloma annulare, epidermal necrolysis, sunburn, photosensitivity, pemphigus, bullous pemphigoid, dermatitis herpetiformis, keratosis pilaris, callouses, corns, ichthyosis, skin ulcers, ischemic necrosis, miliaria, hyperhidrosis, moles, Kaposi's sarcoma, melanoma, malignant melanoma, basal cell carcinoma, squamous cell carcinoma, poison ivy, poison oak, contact dermatitis, atopic dermatitis, rosacea, purpura, moniliasis, candidiasis, baldness, alopecia, Behcet's syndrome, cholesteatoma, Dercum disease, ectodermal dysplasia, gustatory sweating, nail patella syndrome, lupus, hives, hair loss, Hailey-Hailey disease, chemical or thermal skin burns, scleroderma, aging skin, wrinkles, sun spots, necrotizing fasciitis, necrotizing myositis, gangrene, scarring, and vitiligo; wherein the active agent is suitable for treating said disorder or may be selected from the group consisting of chlamydia infection, gonorrhea infection, hepatitis B, herpes, HIV/AIDS, human papillomavirus (HPV), genital warts, bacterial vaginosis, candidiasis, chancroid, granuloma Inguinale, lymphogranuloma venereum, mucopurulent cervicitis (MPC), molluscum contagiosum, nongonococcal urethritis (NGU), trichomoniasis, vulvar disorders, vulvodynia, vulvar pain, yeast infection, vulvar dystrophy, vulvar intraepithelial neoplasia (VIN), contact dermatitis, pelvic inflammation, endometritis, salpingitis, oophoritis, genital cancer, cancer of the cervix, cancer of the vulva, cancer of the vagina, vaginal dryness, dyspareunia, anal and rectal disease, anal abscess/fistula, anal cancer, anal fissure, anal warts, Crohn's disease, hemorrhoids, anal itch, pruritus ani, fecal incontinence, constipation, polyps of the colon and rectum; wherein the active agent is suitable for treating said disorder.

The disorder may be a dermatological disorder, which can be treated by a dicarboxylic acid or dicarboxylic acid ester.

The disorder may be a dermatological disorder, which can be treated by a topical steroid, an immunomodulator or an anti-infective agent.

The disorder may be selected from atopic dermatitis and psoriasis; and the active agent is selected from (i) steroid; and (ii) a combination of steroid and an additional non-steroidal active agent.

The disorder may be selected from psoriasis and atopic dermatitis and the active agent comprises an immunomodulator.

In one or more embodiments there is provided a foamable composition comprising: a liquid dicarboxylic acid ester, said ester having emollient properties; a stabilizer selected from the group consisting of at least one surface-active agent; at least one polymeric agent and mixtures thereof, an active agent, said active agent soluble in or having enhanced penetration due to the dicarboxylic acid; wherein the composition is contained in a pressurized container and further comprises a liquefied hydrocarbon gas propellant at a concentration of about 3% to about 25% by weight of the total composition it is substantially flowable and provides a foam upon release

In one or more embodiments the stabilizer comprises a ether-based or ester-based surfactant.

In one or more embodiments the stabilizer comprises an alkyl-derivatized polymer having polymeric emulsifying properties.

In one or more embodiments the composition is an oil in water emulsion.

In one or more embodiments the dicarboxylic acid ester comprises about or more than 50 wt% of the composition.

In one or more embodiments the active agent is otherwise insoluble or unstable, but is solubilized or stabilized by DCA.

In one or more embodiments the composition is substantially free of water In one or more embodiments the composition is in a non foam state.

All % values are provided on a weight (w/w) basis.

## Dicarboxylic acid and esters thereof

In an embodiment of the present invention, the organic carrier comprises an ester of a dicarboxylic acid. In the context of the present invention, a dicarboxylic acid is an organic material, having two carboxylic acid moieties on its carbon atom skeleton. They have the general molecular formula $HOOC\text{-}(CH_2)_n\text{-}COOH$.

Non limiting examples of some elementary dicarboxylic acids (DCA's) are succinic acid, glutaric acid, adipic acid, pimelic acid, suberic acid, azelaic acid, sebacic acid, phthalic acid, isophthalic acid, terephthalic acid.

In an embodiment of the present invention, the dicarboxylic acid is a short-chain dicarboxylic acid. The simplest Short-chain dicarboxylic acid are oxalic acid (n=0), malonic acid (n=1), succinic acid (n=2) and glutaric acid (n=3).

Additional members of dicarboxylic acid group are derived from natural products or from synthesis, having "n" value from 4 up to 21. In one or more embodiments of the present invention, the dicarboxylic acid is selected from the group consisting of adipic acid (hexanedioic acid; n=4), pimelic acid (heptanedioic acid; n=5), suberic acid (octanedioic acid; n=6), azelaic acid (nonanedioic acid; n=7), sebacic acid (decanedioic acid; n=8) and dodecanedioic acid (n=10).

In an additional embodiment, the dicarboxylic acid contains 1 0 to 32 carbon atoms in their carbon atom skeleton, such as brassylic acid (n=11), thapsic acid (n=14), 14-methylnonacosanedioic acid (C29) and 14,15-dimethyltriacontanedioic acid (C30).

The carbon atom skeleton of the dicarboxylic acid can be saturated or unsaturated, such as in the case of maleic acid and fumaric acid.

In general terms non-esterified dicarboxylic acids are usually solid at ambient temperature. Non limiting examples of solid DCA's are oxalic, malonic glutaric, sebacic , phthalic and azaleic acid. Similarly, in general terms DCA's with short carbon chain skeleton are water soluble, such as oxalic, malonic, and succinic acid. Longer chain DCA's like adipic acid and having up to 10 carbon atoms in the carbon chain are slightly soluble in water. Also non "simple" DCA's are generally solid at ambient temperature , insoluble in water, and are usually more oil soluble than their parent DCA's

An ester of a dicarboxylic acid is a chemical compound produced by the reaction between a dicarboxylic acid and at least one alcohol, with the elimination of a molecule of water. The reaction of a dicarboxylic acid with one alcohol molecule results in a mono ester of a dicarboxylic acid. The reaction of a dicarboxylic acid with two alcohol molecules results in a diester of the dicarboxylic acid.

DCA esters are typically hydrophobic and generally insoluble in water. Most simple esters of DCA are liquid. By simple it is meant that the alcohol moiety linked to the DCA is a straight or branched alkyl chain. Examples of liquid simple diesters are dimethyl phthalate, diethyl phthalate, dibutyl phthalate, diethyl sebacate, dibutyl sebacate, and diisopropyl adipate. Aromatic diesters of pthalic, isopthalic and therephalic acids are in the range of slightly soluble to insoluble.

The alcohol molecule, to be linked to the dicarboxylic acid, can be selected from the group of an alkyl an aryl alcohol. Exemplary alcohol, suitable according to the present invention include methyl alcohol, ethyl alcohol, propyl alcohol, isopropyl alcohol, butyl alcohol, isobutyl alcohol, t-butyl alcohol, pentyl alcohol, hexyl alcohol, octyl alcohol, decyl alcohol, capryl alcohol, phenol, benzyl alcohol and the like.

In one or more embodiments, the alcohol is a biologically active alcohol. In an embodiment, biologically active alcohol possesses keratolytic activities. Examples of keratolytically active alcohol suitable according to the present invention include ortho-, meta- and para-hydroxyalkylbenzoate, salicylic acid, ortho-, meta-, and para-dihydroxybenzene, ortho-, meta-, and para-hydroxytoluene, alpha-hydroxy acid, retinol, and derivatives thereof such as provided in U.S. Pat. 6,180,669, which is incorporated herein by reference. In another embodiment, the biologically active alcohol is selected from the group consisting of steroidal hormones, steroidal anti-inflammatory agents, vitamin E and vitamin D, such as provided in U.S. Pat. Appl. 20040191196, which is incorporated herein by reference.

In an embodiment of the present invention, the azelaic acid is incorporated in the foamable composition in a safe and effective amount. The term "safe and effective" means an amount of an active agent that exerts a therapeutic effect on a specific disorder, without causing adverse effects that may prohibit the use of said active agent in the treatment of said disorder. The azelaic acid can be incorporated in the foamable composition of the present invention in a concentration between about 0.1% and about 25%, more preferably between about 1% and about 20%.

In an embodiment of the present invention, the dicarboxylic acid is azelaic acid, and its concentration in the composition is between 5% and 25%, or between 10% and 20%.

In one or more embodiment, the azelaic acid is present in the composition in an ionized state. The first and second pKa values for a dicarboxylic acid are different from one another. Depending on the pH of the composition and the specific first and second pKa of the dicarboxylic acid, said dicarboxylic acid can be non-ionized (both carboxy groups are in their acid state); semi-ionized (one carboxy group is in an acid state and the second is in an anionic state); or doubly-ionized, wherein both carboxy groups are anionic. For example, in the case of azelaic acid the first pKa is about 4.5 and the second pKa is about 5.3. Therefore, if the pH of the composition is below 4.5, the azelaic acid is non-ionized; between about 4.5 and about 5.3, it is mostly semi-ionized and at a pH above 5.3, the azelaic acid is mostly doubly-ionized.

The ionization state of the dicarboxylic acid has influence on its therapeutic potential. On one hand, if the dicarboxylic acid is doubly anionic, its penetration into the skin will be very low, due to the lipophilic nature of the skin. On the other hand, the non-ionic state is available at very low (acidic) pH values, which can cause skin irritation.

Hence, in one or more embodiments, the pH of the composition is adjusted to a value between the first and second pKa values of the dicarboxylic acid. For example, in the case of azelaic acid, the pH is adjusted in the range from about 2.0 to about 4.5, preferably in the range from about 3.0 to about 4.5. Thus, in an embodiment of the present invention, the pH of the composition is adjusted in the range from about 4.0 to about 6.0, preferably in the range from about 4.5 to about 5.3.

Dicarboxylic acid esters are considered excellent emollients and their inclusion in a composition which is intended for topical application contributes to the overall improvement of skin condition. Emollient dicarboxylic esters typically include an alkyl alcohol moiety, wherein said alkyl alcohol has a carbon chain of at least one or two or more carbon atoms. In certain embodiments, the alkyl alcohol is a branched alkyl, such as isopropyl alcohol; and in other embodiments the alkyl alcohol has a long carbon backbone, e.g., a carbon chain length of 6-18.

Dicarboxylic acid esters can be complex substances. One example is TU 2100 (Nonanedioic acid, bis[(2-(ethoxycarbonyl)phenyl] ester). It is also known as Azelaoyl di(ethyl salicylate) and has a CAS Registry Number: [207972-39-2] and is a solid. TU-2100 is a "non-simple" diester; with a high molecular weight, and a melting point of 34-36, which is relatively low with reference to its molecular weight.

Non-limiting examples of emollient dicarboxylic acid esters include diisobutyl adipate, diisopropyl adipate, diisopropyl sebacate, diisosteary dimer dilinoleate, diisostearyl fumerate, diisopropyl dimerate, diethyl adipate, diethyl sebacate, diethylhexyl adipate, diethylhexyl malate, dioctyl malate, diethyl succinate, and dioctyl sebacate. Other dicarboxylic acid esters are dimethyl phthalate, diethyl phthalate, diethyl sebacate, diisopropyl dimerate, dibutyl sebacate, dibutyl phthalate and dioctyl phthalate. Additionally dicarboxylic acid esters are capable of solubilizing active components which are difficult to dissolve by other oils. Furthermore, certain dicarboxylic acid esters, such as diisopropyl adipate and dimethyl sebacate are known to enhance the skin penetration of active agents. Hence in an embodiment of the present invention, the dicarboxylic acid ester is incorporated in the foamable composition in an amount, suitable to exert its emollient effect, solubilizing effect or skin penetration enhancing effect. In one or more embodiments, the dicarboxylic acid ester is incorporated in the foamable composition of the present invention in a concentration between about 0.1 % and about 30%, more preferably between about 1% and about 25%.

In one embodiment, the dicarboxylic acid ester is diisopropyl adipate (DISPA), in an amount between about 0.1 % and about 30%, or about 1% and about 25%.

As can be appreciated by the discussion above, there is a varied range of dicarboxylic acids and esters; some are soild, some are liquid, some are water soluble, some are slightly soluble and others are insoluble in water. There is also a varied range of functions and physical properties. Some are active agents and others are solvents and some are penetration enhancers. The challenges of making a uniform solution of solid DCA's wihout crystal formation or precipitation or a uniform suspension of insoluble agent, or using a DCA to solubilise a substance which is otherwise insoluble or as an emollient or as a penetration enhancer or more than one of them are as varied as their different natures and properties as may be appreciated by a man of the art. In other words each agent has its own properties and challenges which are interrelated to the objectives and other ingredients of the formulation

The sensory properties of foams containing a dicarboxylic acid or a dicarboxylic acid ester are favorable, as revealed by consumer panel tests.

**Foam adjuvant**

Optionally, the foamable vehicle further includes a foam adjuvant selected from the group consisting of a fatty alcohol having 15 or more carbons in their carbon chain; a fatty acid having 16 or more carbons in their carbon chain; fatty alcohols, derived from beeswax and including a mixture of alcohols, a majority of which has at least 20 carbon atoms in their carbon chain; a fatty alcohol having at least one double bond; a fatty acid having at least one double bond; a branched fatty alcohol; a branched fatty acid and a fatty acid substituted with a hydroxyl group.

**Additional organic carrier**

Optionally, the foamable vehicle further includes at least one additional organic carrier selected from the group consisting of a hydrophobic organic carrier, an emollient and mixtures thereof, at a concentration of about 2% to about 50% by weight. The hydrophobic solvent and/or the emollient can be selected from the group consisting of mineral oil, triglycerides, capric/caprylic triglyceride, alkyl esters of fatty acids such as isopropyl palmitate, isopropyl isostearate, octyl palmitate, cetyl lactate, cetyl ricinoleate, tocopheryl acetate, acetylated lanolin alcohol, cetyl acetate, phenyl trimethicone, glyceryl oleate, tocopheryl linoleate, wheat germ glycerides, arachidyl propionate, myristyl lactate, decyl oleate, ricinoleate, isopropyl lanolate, pentaerythrityl tetrastearate, neopentylglycol dicaprylate/dicaprate, isononyl isononanoate, isotridecyl isononanoate, myristyl myristate, triisocetyl citrate, octyl dodecanol, maleated soybean oil, unsaturated or polyunsaturated oils, such as olive oil, corn oil, soybean oil, canola oil, cottonseed oil, coconut oil, sesame oil, sunflower oil, borage seed oil, syzigium aromaticum oil, hempseed oil, herring oil, cod-liver oil, salmon oil, flaxseed oil, wheat germ oil, evening primrose oils; essential oils; and silicone oils, such as dimethicone, cyclomethicone, polyalkyl siloxane, polyaryl siloxane, polyalkylaryl siloxane, a polyether siloxane copolymer and a poly(dimethylsiloxane)-(diphenyl-siloxane) copolymer.

According to a preferred embodiment, the organic carrier does not contain petrolatum, which is also termed "white petrolatum" and "Vaseline". Petrolatum often forms an impermeable occlusive barrier, so that metabolic products and excreta from damaged tissue are not easily removed or drained away. Furthermore, it is difficult for the active drug dissolved in the carrier to pass through the white petrolatum barrier layer into the treated tissue, so the efficacy of the drug is reduced. An additional disadvantage of petroleum jelly-based products relates to the greasy feeling left following their topical application onto the skin, mucosal membranes and wounds causing inconvenience to the user, thereby decreasing treatment compliance.

**Polymeric agent**

The composition of the present invention contains a polymeric agent selected from the group consisting of a bioadhesive agent, a gelling agent, a film forming agent and a phase change agent. A polymeric agent enhances the creation of foam having fine bubble structure, which does not readily collapse upon release from the pressurized aerosol can. The polymeric agent serves to stabilize the foam composition and to control drug residence in the target organ.

Exemplary polymeric agents include, in a non-limiting manner, naturally-occurring polymeric materials, such as locust bean gum, sodium alginate, sodium caseinate, egg albumin, gelatin agar, carrageenin gum, sodium alginate, xanthan gum, quince seed extract, tragacanth gum, guar gum, cationic guars, hydroxypropyl guar gum, starch, amine-bearing polymers such as chitosan; acidic polymers obtainable from natural sources, such as alginic acid and hyaluronic acid; chemically modified starches and the like, carboxyvinyl polymers, polyvinylpyrrolidone, polyvinyl alcohol, polyacrylic acid polymers, polymethacrylic acid polymers, polyvinyl acetate polymers, polyvinyl chloride polymers, polyvinylidene chloride polymers and the like.

Additional exemplary polymeric agents include semi-synthetic polymeric materials such as cellulose ethers, such as methylcellulose, hydroxypropyl cellulose, hydroxypropyl methylcellulose, hydroxyethyl cellulose, hydroxy propylmethyl cellulose, methylhydroxyethylcellulose, methylhydroxypropylcellulose, hydroxyethylcarboxymethylcellulose, carboxymethyl cellulose, carboxymethylhydroxyethylcellulose, and cationic celluloses, carbomer (homopolymer of acrylic acid is crosslinked with an allyl ether pentaerythritol, an allyl ether of sucrose, or an allyl ether of propylene, such as Carbopol® 934, Carbopol® 940, Carbopo® 941, Carbopol® 980 and Carbopol® 981, pemulen and aluminum starch octenylsuccinate (ASOS). Polyethylene glycol, having molecular weight of 1000 or more (e.g., PEG 1,000, PEG 4,000, PEG 6,000 and PEG 10,000) also have gelling capacity and while they are considered herein as "secondary polar solvents", as detailed herein, they are also considered polymeric agents.

In one or more embodiments the polymeric agents have emulsifying properties. In certain preferred embodiments the polymeric agent is a derivatized hydrophilic polymer with hydrophobic alkyl moieties. Other types that may also a similar stabilizing effect are silicone copolymers and derivatized starch ASOS.

Mixtures of the above polymeric agents are contemplated.

The concentration of the polymeric agent should be selected so that the composition, after filling into aerosol canisters, is flowable, and can be shaken in the canister. In one or more embodiments, the concentration of the polymeric agent is selected such that the viscosity of the composition, prior to filling of the composition into aerosol canisters, is less than 12,000 CPs, and more preferably, less than 10,000 CPs.

**Surface Active Agent**

The composition of the present invention further contains a surface-active agent. Surface-active agents (also termed "surfactants") include any agent linking oil and water in the composition, in the form of emulsion. A surfactant's hydrophilic/lipophilic balance (HLB) describes the emulsifier's affinity toward water or oil. HLB is defined for non-ionic surfactants. The HLB scale ranges from 1 (totally lipophilic) to 20 (totally hydrophilic), with 10 representing an equal balance of both characteristics. Lipophilic emulsifiers form water-in-oil (w/o) emulsions; hydrophilic surfactants form oil-in-water (o/w) emulsions. The HLB of a blend of two emulsifiers equals the weight fraction of emulsifier A times its HLB value plus the weight fraction of emulsifier B times its HLB value (weighted average). In many cases a single surfactant may suffice. In other cases a combination of two or more surfactants is desired. Reference to a surfactant in the specification can also apply to a combination of surfactants or a surfactant system. As will be appreciated by a person skilled in the art which surfactant or surfactant system is more appropriate is related to the vehicle and intended purpose. In general terms a combination of surfactants is usually preferable where the vehicle is an emulsion. In an emulsion environment a combination of surfactants can be significant in producing breakable forms of good quality. It has been further discovered that the generally thought considerations for HLB values for selecting a surfactant or sufactant combination are not always binding for emulsions and that good quality foams can be produced with a surfactant or surfactant combination both where the HLB values are in or towards the lipophilic side of the scale and where the HLB values are in or towards the hydrophilic side of the scale. Surfactants also play a role in foam formation where the foamable formulation is a single phase composition.

According to one or more embodiments the composition contains a single surface active agent having an HLB value between about 2 and 9, or more than one surface active agent and the weighted average of their HLB values is between about 2 and about 9. Lower HLB values may in certain embodiments be more applicable to water in oil emulsions.

According to one or more embodiments the composition contains a single surface active agent having an HLB value between about 7 and 14, or more than one surface active agent and the weighted average of their HLB values is between about 7 and about 14. Mid range HLB values may in certain embodiments be more suitable for oil in water emulsions.

According to one or more other embodiments the composition contains a single surface active agent having an HLB value between about 9 and about 19, or more than one surface active agent and the weighted average of their HLB values is between about 9 and about 19. In a waterless or substantially waterless environment a wide range of HLB values may be suitable.

Preferably, the composition of the present invention contains a non-ionic surfactant. Nonlimiting examples of possible non-ionic surfactants include a polysorbate, polyoxyethylene (20) sorbitan monostearate, polyoxyethylene (20) sorbitan monooleate, a polyoxyethylene fatty acid ester, Myrj 45, Myrj 49, Myrj 52 and Myrj 59; a polyoxyethylene alkyl ether, polyoxyethylene cetyl ether, polyoxyethylene palmityl ether, polyethylene oxide hexadecyl ether, polyethylene glycol cetyl ether, steareths such as steareth 2, brij 21, brij 721, brij 38, brij 52, brij 56 and brij W1, a sucrose ester, a partial ester of sorbitol and its anhydrides, sorbitan monolaurate, sorbitan monolaurate, a monoglyceride, a diglyceride, isoceteth-20 and mono-, di- and tri-esters of sucrose with fatty acids. In certain embodiments, suitable sucrose esters include those having high monoester content, which have higher HLB values.

In certain embodiments with DCA esters as emollient, surfactants are selected which can provide a close packed sufacant layer separating the oil and water phases. To achieve such objectives combinations of at least two surfactants are selected. Preferrably, they should be complex emulgators and more preferably they should both be of a similar molecular type. For example, a pair of ethers like steareth 2 and steareth 21, or a pair of esters for example, PEG-40 stearate and polysorbate 80. In certain circumstances POE esters cannot be used and a combination of sorbitan laurate and sorbitan stearate or a combination of sucrose stearic acid ester mixtures and sodium laurate may be used. All these combinations due to heir versatility and strength may also be used satisfactorily and effectively with solutions of DCA's and with solid/crystalline suspensions, although the amounts and proportion may be varied according to the formulation and its objectives as will be appreciated by a man of the art.

It has been discovered also that by using a derivatized hydrophilic polymer with hydrophobic alkyl moieties as a polymeric emulsifier such as pemulen it is possible to stabilize the emulsion better about or at the region of phase reversal tension. Other types of derivatized polymers like silicone copolymers, derivatized starch [Aluminum Starch Octenylsuccinate (ASOS)] / [DRY-FLO AF Starch], and derivatized dexrin may also a similar stabilizing effect.

A series of dextrin derivative surfactants prepared by the reaction of the propylene glycol polyglucosides with a hydrophobic oxirane-containing material of the glycidyl ether are highly biodegradable. [Hong-Rong Wang and Keng-Ming Chen, Colloids and Surfaces A: Physicochemical and Engineering Aspects Volume 281, Issues 1-3, 15 June 2006, Pages 190-193].

Non-limiting examples of non-ionic surfactants that have HLB of about 7 to about 12 include steareth 2 (HLB~4.9); glyceryl monostearate/PEG 100 stearate (Av HLB~11.2); stearate laureth 4 (HLB-9.7) and cetomacrogol ether (e.g., polyethylene glycol 1000 monocetyl ether).

Non-limiting examples of preferred surfactants, which have a HLB of 4-19 are set out in the Table below:

| Surfactant | HLB |
|---|---|
| steareth 2 | ~4.9 |
| glyceryl monostearate/PEG 100 stearate | Av ~11.2 |
| Glyceryl stearate | ~4 |
| steareth-21 | ~15.5 |
| peg 40 stearate | ~16.9 |
| polysorbate 80 | ~15 |
| sorbitan stearate | ~4.7 |
| laureth 4 | ~9.7 |
| sorbitan monooleate (span 80) | ~4.3 |
| ceteareth 20 | ~15.7 |
| steareth 20 | ~15.3 |
| ceteth 20 | ~15.7 |
| macrogol cetostearyl ether | ~15.7 |
| ceteth 2 (Lipocol C-2) | ~5.3 |
| PEG-30 Dipolyhydroxystearate | ~5.5 |
| sucrose distearate (Sisterna SP30) | ~6 |
| polyoxyethylene (100) stearate | ~18.8 |

More exemplary stabilizing surfactants which may be suitable for use in the present invention are found below.

### PEG-Fatty Acid Monoester Surfactants

| Chemical name | Product example name | HLB |
|---|---|---|
| PEG-30 stearate | Myrj 51 | >10 |
| PEG-40 laurate | Crodet L40 (Croda) | 17.9 |
| PEG-40 oleate | Crodet 040 (Croda) | 17.4 |
| PEG-45 stearate | Nikkol MYS-45 (Nikko) | 18 |
| PEG-50 stearate | Myrj 53 | >10 |
| PEG-100 stearate | Myrj 59, Arlacel 165 (ICI) | 19 |

**PEG-Fatty Acid Diester Surfactants:**

| Chemical name | Product example name | HLB |
|---|---|---|
| PEG-4 dilaurate | Mapeg .RTM. 200 DL (PPG), Kessco .RTM.PEG 200 DL (Stepan), LIPOPEG 2-DL (Lipo Chem.) | 7 |
| PEG-4 | distearate Kessco .RTM. 200 DS (Stepan.sub) | 5 |
| PEG-32 dioleate | Kessco .RTM. PEG 1540 DO (Stepan) | 15 |
| PEG-400 dioleate | Cithrol 4DO series (Croda) | >10 |
| PEG-400 disterate | Cithrol 4DS series (Croda) | >10 |
| PEG-20 glyceryl oleate | Tagat .RTM. O (Goldschmidt) | >10 |

**Transesterification Products of Oils and Alcohols**

| Chemical name | Product example name | HLB |
|---|---|---|
| PEG-30 castor oil | Emalex C-30 (Nihon Emulsion) | 11 |
| PEG-40 hydrogenated castor oil | Cremophor RH 40 (BASF), Croduret (Croda), Emulgin HRE 40 (Henkel) | 13 |

**Polyglycerized Fatty Acids, such as:**

| Chemical name | Product example name | LB |
|---|---|---|
| Polyglyceryl-6 dioleate | Caprol .RTM. 6G20 (ABITEC); PGO-62 (Calgene), PLUROL OLEIQUE CC 497 (Gattefosse)Hodag | 8.5 |

**PEG-Sorbitan Fatty Acid Esters**

| Chemical name | Product example name | HLB |
|---|---|---|
| PEG-20 sorbitan monolaurate | Tween-20 (Atlas/ICI), Crillet 1 (Croda), DACOL MLS 20 (Condea) | 17 |
| PEG-20 sorbitan Monopalmitate | Tween 40 (Atlas/ICI), Crillet 2 (Croda) | 16 |
| PEG-20 sorbitan monostearate | Tween-60 (Atlas/ICI), Crillet 3 (Croda) | 15 |
| PEG-20 sorbitan monooleate | Tween-80 (Atlas/ICI), Crillet 4 (Croda) | 15 |

**Polyethylene Glycol Alkyl Ethers**

| Chemical name | Product example name | HLB |
|---|---|---|
| PEG-2 oleyl ether | oleth-2 Brij 92/93 (Atlas/ICI) | 4.9 |
| PEG-3 oleyl ether | oleth-3 Volpo 3 (Croda) | <10 |
| PEG-5 oleyl ether | oleth-5 Volpo 5 (Croda) | <10 |
| PEG-10 oleyl ether | oleth-10 Volpo 10 (Croda), Brij 96/97 (Atlas/ICI) | 12 |
| PEG-20 oleyl ether | oleth-20 Volpo 20 (Croda), Brij 98/99 (Atlas/ICI) | 15 |
| PEG-4 lauryl ether | laureth-4Brij 30 (Atlas/ICI) | 9.7 |
| PEG-23 lauryl ether | laureth-23Brij 35 (At)as/ICI) | 17 |
| PEG-10 stearyl ether | Brij 76 (ICI) | 12 |
| PEG-2 cetyl ether | Brij 52 (ICI) | 5.3 |

**Sugar Ester Surfactants**

| Chemical name | Product example name | HLB |
|---|---|---|
| Sucrose distearate | Sisterna SP50, Surfope 1811 | 11 |

**Sorbitan Fatty Acid Ester Surfactants**

| Chemical name | Product example name | HLB |
|---|---|---|
| Sorbitan monolaurate | Span-20 (Atlas/ICI), Crill 1 (Croda), Arlacel 20 (ICI) | 8.6 |
| Sorbitan monopalmitate | Span-40 (Atlas/ICI), Crill 2 (Croda), Nikkol SP-10 (Nikko) | 6.7 |
| Sorbitan monooleate | Span-80 (Atlas/ICI), Crill 4 (Croda), Crill 50 (Croda) | 4.3 |
| Sorbitan monostearate | Span-60 (Atlas/ICI), Crill 3 (Croda), Nikkol SS-10 (Nikko) | 4.7 |

In one or more embodiments the surface active agent is a complex emulgator in which the combination of two or more surface active agents can be more effective than a single surfactant and provides a more stable emulsion or improved foam quality than a single surfactant. For example and by way of non-limiting explanation it has been found that by choosing say two surfactants, one hydrophobic and the other hydrophilic the combination can produce a more stable emulsion than a single surfactant. Preferably, the complex emulgator comprises a combination of surfactants wherein there is a difference of about 4 or more units between the HLB values of the two surfactants or there is a significant difference in the chemical nature or structure of the two or more surfactants.

Specific non limiting examples of surfactant systems are, combinations of polyoxyethylene alkyl ethers, such as Brij 59 / Brij10; Brij 52 / Brij 10; Steareth 2 / Steareth 20; Steareth 2 / Steareth 21 (Brij 72 / Brij 721); combinations of polyoxyethylene stearates such as Myrj 52 / Myrj 59; combinations of sucrose esters, such as Surphope 1816 / Surphope 1807; combinations of sorbitan esters, such as Span 20 / Span 80; Span 20 / Span 60; combinations of sucrose esters and sorbitan esters, such as Surphope 1811 and Span 60; combinations of liquid polysorbate detergents and PEG compounds, such as Tween 80 / PEG-40 stearate; methyl glucaso sequistearate; polymeric emulsifiers, such as Permulen (TRI or TR2); liquid crystal systems, such as Arlatone (2121), Stepan (Mild RM1), Nikomulese (41) and Montanov (68) and the like.

In certain embodiments the surfactant is preferably one or more of the following: a combination of steareth-2 and steareth-21 on their own or in combination with glyceryl monostearate (GMS); in certain other embodiments the surfactant is a combination of polysorbate 80 and PEG-40 stearate. In certain other embodiments the surfactant is a combination of glyceryl monostearate/PEG 100 stearate. In certain other embodiments the surfactant is a combination of two or more of stearate 21, PEG 40 stearate, and polysorbate 80. In certain orher embodiments the surfactant is a combination of two or more of laureth 4, span80, and polysorbate 80. In certain other embodiments the surfactant is a combination of two or more of GMS and ceteareth. In certain other embodiments the surfactant is a combination of two or more of steareth 21, ceteareth 20, ceteth 2 and laureth 4. In certain other embodiments the surfactant is a combination of ceteareth 20 and polysorbate 40 stearate. In certain orther embodiments the surfactant is a combination of span 60 and GMS.

In certain other embodiments the surfactant is one or more of sucrose stearic acid esters, sorbitan laureth, and sorbitan stearate.

In one or more embodiments the stability of the composition can be improved when a combination of at least one non-ionic surfactant having HLB of less than 9 and at least one non-ionic surfactant having HLB of equal or more than 9 is employed. The ratio between the at least one non-ionic surfactant having HLB of less than 9 and the at least one non-ionic surfactant having HLB of equal or more than 9, is between 1:8 and 8:1, or at a ratio of 4:1 to 1:4. The resultant HLB of such a blend of at least two emulsifiers is preferably between about 9 and about 14.

Thus, in an exemplary embodiment, a combination of at least one non-ionic surfactant having HLB of less than 9 and at least one non-ionic surfactant having HLB of equal or more than 9 is employed, at a ratio of between 1:8 and 8:1, or at a ratio of 4:1 to 1:4, wherein the HLB of the combination of emulsifiers is preferably between about 5 and about 18.

In certain cases, the surface active agent is selected from the group of cationic, zwitterionic, amphoteric and ampholytic surfactants, such as sodium methyl cocoyl taurate, sodium methyl oleoyl taurate, sodium lauryl sulfate, triethanolamine lauryl sulfate and betaines.

Many amphiphilic molecules can show lyotropic liquid-crystalline phase sequences depending on the volume balances between the hydrophilic part and hydrophobic part. These structures are formed through the micro-phase segregation of two incompatible components on a nanometer scale. Soap is an everyday example of a lyotropic liquid crystal. Certain types of surfactants tend to form lyotropic liquid crystals in emulsions interface (oil-in-water) and exert a stabilizing effect.

In one or more embodiments the surfactant is a surfactant or surfactant combination is capable of or which tends to form liquid crystals. Surfactants which tend to form liquid crystals may improve the quality of foams. Non limiting examples of surfactants with postulated tendency to form interfacial liquid crystals are: phospholipids, alkyl glucosides, sucrose esters, sorbitan esters.

In one or more embodiments the at least one surface active agent is liquid.

In one or more embodiments the at least one surface active agent is solid, semi solid or waxy.

It should be noted that HLB values may not be so applicable to non ionic surfactants, for example, with liquid crystals or with silicones. Also HLB values may be of lesser significance in a waterless or substantially non-aqueous environment.

In one or more embodiments the surfactant can be, a surfactant system comprising of a surfactant and a co surfactant, a waxy emulsifier, a liquid crystal emulsifier, an emulsifier which is solid or semi solid at room temperature and pressure, or combinations of two or more agents in an appropriate proportion as will be appreciated a person skilled in the art. Where a solid or semi solid emulsifier combination is used it can also comprise a solid or semi solid emulsifier and a liquid emulsifier.

In one or more embodiments of the present invention, the surface-active agent includes at least one non-ionic surfactant. Ionic surfactants are known to be irritants. Therefore, non-ionic surfactants are preferred in applications including sensitive tissue such as found in most mucosal tissues, especially when they are infected or inflamed. Non-ionic surfactants alone can provide formulations and foams of good or excellent quality in the carriers and compositions of the present invention.

Thus, in a preferred embodiment, the surface active agent, the composition contains a non-ionic surfactant. In another preferred embodiment the composition includes a mixture of non-ionic surfactants as the sole surface active agent. Yet, in additional embodiments, the foamable composition includes a mixture of at least one non-ionic surfactant and at least one ionic surfactant in a ratio in the range of about 100:1 to 6:1. In one or more embodiments, the non-ionic to ionic surfactant ratio is greater than about 6:1, or greater than about 8:1; or greater than about 14:1, or greater than about 16:1, or greater than about 20:1. In further embodiments, surface active agent comprises a combination of a non-ionic surfactant and an ionic surfactant, at a ratio of between 1:1 and 20:1

In one or more embodiments of the present invention, a combination of a non-ionic surfactant and an ionic surfactant (such as sodium lauryl sulphate and cocamidopropylbetaine) is employed, at a ratio of between 1:1 and 20:1, or at a ratio of 4:1 to 10:1; for example, about 1:1, about 4:1, about 8:1, about 12:1, about 16:1 and about 20:1 or at a ratio of 4:1 to 10:1, for example, about 4:1, about 6:1, about 8:1 and about 10:1.

In selecting a suitable surfactant or combination thereof it should be borne in mind that the upper amount of surfactant that may be used may be limited by the shakability of the composition. If the surfactant is non liquid, it can make the formulation to viscous or solid. This can be particularly significant if the formulation has high molecular weight, e.g., a high molecular weight PEG or polymeric agents or petroleum or if the surfactants are large. Solvents and polymeric agents which have high molecular weight and are very viscous or solid or waxy (e.g., Peg 1500, 2000, etc. or petrolatum) can exacerbate the effect of a waxy or solid surfactant on shakability or flowability. In general terms, as the amount of non-liquid surfactant is increased the shakability of the formulation reduces until a limitation point is reached where the formulation becomes non shakable and unsuitable. Thus in one embodiment, an effective amount of surfactant may be used provided the formulation remains shakable. In other certain exceptional embodiments the upper limit may be determined by flowability such as in circumstances where the composition is marginally or apparently non-shakable. The formulation is sufficiently flowable to be able to flow through an actuator valve and be released and still expand to form a good quality foam.

In certain embodiments of the present invention the amount of surfactant or combination of surfactants is between about 0.05% to about 20%; between about 0.05% to about 15% or between about 0.05% to about 10%. In a preferred embodiment the concentration of surface active agent is between about 0.2% and about 8%. In a more preferred embodiment the concentration of surface active agent is between about 1 % and about 6%.

In some embodiments, it is desirable that the surface active agent does not contain a polyoxyethylene (POE) moiety, such as polysorbate surfactants, POE fatty acid esters, and POE alkyl ethers, because the active agent is incompatible with such surface active agents. For example, the active agent pimecrolimus is not stable the presence of POE moieties, yet benefits greatly from the use of dicarboxylic esters as penetration enhancers. In such cases, alternative surface active agents are employed. In an exemplary manner, POE - free surfactants include non-ethoxylated sorbitan esters, such as sorbitan monopalmitate, sorbitan monostearate, sorbitan tristearate, sorbitan monooleate, sorbitan trioleate, sorbitan monolaurate and sorbitan sesquioleate; glycerol fatty acid esters, such as glycerol monostearate and glycerol monooleate; mono-, di- and tri-esters of sucrose with fatty acids (sucrose esters), sucrose stearate, sucrose distearate sucrose palmitate and sucrose laurate; and alkyl polyglycosides, such as lauryl diglucoside.

If the composition as formulated is a substantially non shakable composition it is nevertheless possible as an exception in the scope of the present invention for the formulation to be flowable to a sufficient degree to be able to flow through an actuator valve and be released and still expand to form a good quality foam. This surprising and unusual exception may be due one or more of a number of factors such as the high viscosity, the softness, the lack of crystals, the pseudoplastic or semi pseudo plastic nature of the composition and the dissolution of the propellant into the composition.

In one or more embodiments of the present invention, the surface-active agent includes mono-, di- and tri-esters of sucrose with fatty acids (sucrose esters), prepared from sucrose and esters of fatty acids or by extraction from sucro-glycerides. Suitable sucrose esters include those having high monoester content, which have higher HLB values.

Phase inversion and tension

Phase inversion is a factor in the preparation and stabilization of emulsions and can be both an aid and a detriment. Phase inversion involves the change of emulsion type from o/w to w/o or vice versa. Prior to phase inversion occurring there is a tension in the emulsion which if destabilized or driven will lead to phase inversion and if controlled or ameliorated or dissipated will result in a more stable emulsion. The occurrence of phase inversion during preparation can be a sign of instability. If controlled, it can result in a finer product but if due to other factors after the the emulsion was prepared it can cause problems. Inversion can occur by for example adding calcium chloride to an o/w emulsion stabilized with sodium stearate to form calcium stearate. Inversion can also occur as the product of changes to the phase- volume ratio. For example if a small amount of water is added to surfactant mixed with oil and agitated aw/o emulsion is formed. As the amount of water added is gradually increased a point will be reached where the water and emulsifier envlop the oil as small droplets to form an o/w emulsion. The amount of each ingredient including the surfactants will have their part to play in the phenomenum.

Substantially Alcohol-Free

According to one or more embodiments, the foamable composition is substantially alcohol-free, i.e., free of short chain alcohols. Short chain alcohols, having up to 5 carbon atoms in their carbon chain skeleton and one hydroxyl group, such as ethanol, propanol, isopropanol, butaneol, iso-butaneol, t-butaneol and pentanol, are considered less desirable solvents or polar solvents due to their skin-irritating effect. Thus, the composition is substantially alcohol-free and includes less than about 5% final concentration of lower alcohols, preferably less than about 2%, more preferably less than about 1%.

Substantially Non Aqueous

In certain cases, the active agent degrades in the presence of water, and therefore, in such cases the presence of water in the composition is not desirable. Thus, in certain preferred embodiments, the composition is substantially non-aqueous. The term "substantially non-aqueous" or "substantially waterless" is intended to indicate that the composition has a water content below about 5%, preferably below about 2%, such as below about 1.5%. In certain other preferred embodiments the composition is non aqueous or waterless.

By non-aqueous or waterless is meant that the composition contains no or substantially no, free or unassociated or absorbed water. It will be understood by a person of the art that the waterless solvents and substances miscible with them of the present invention can be hydrophilic and can contain water in an associated or unfree or absorbed form and may absorb water from the atmosphere and the ability to do so is its hygroscopic water capacity. It is intended that essentially non-aqueous formulations are included within its scope such that the formulations may have present a small amount of water. In some embodiments the composition ingredients are pretreated to reduce, remove or eliminate any residual or associated or absorbed water.

Shakability

'Shakability' means that the composition contains some or sufficient flow to allow the composition to be mixed or remixed on shaking. That is, it has fluid or semi fluid properties. In some very limited cases possibly aided by the presence of silicone it may exceptionally be possible to have a foamable composition which is flowable but not apparently shakable.

Breakability

A breakable foam is one that is thermally stable, yet breaks under shear force.
The breakable foam of the present invention is not "quick breaking", i.e., it does not readily collapse upon exposure to body temperature environment. Shear-force breakability of the foam is clearly advantageous over thermally induced breakability, since it allows comfortable application and well directed administration to the target area.

**Modulating Agent**

The term modulating agent is used to describe an agent which can improve the stability of or stabilize a foamable carrier or composition and or an active agent by modulating the effect of a substance or residue present in the carrier or composition.

In one or more embodiments the modulating agent is used in a water in oil or oil in water emulsion. In one or more other embodiments the modulating agent is used in a unique waterless emulsion.

In certain embodiments the substance or residue may for example be acidic or basic and potentially alter pH in an emulsion environment or it may be one or more metal ions which may act as a potential catalyst in an emulsion environment.

In certain other embodiments the substance or residue may for example be acidic or basic and potentially alter an artificial pH in a waterless or substantially non aqueous environment or it may be one or more metal ions which may act as a potential catalyst in a waterless or substantially non aqueous environment.

In one or more embodiments the modulating agent is used to describe an agent which can affect pH in an aqueous solution. The agent can be any of the known buffering systems used in pharmaceutical or cosmetic formulations as would be appreciated by a man of the art. It can also be an organic acid, a carboxylic acid, a fatty acid an amino acid, an aromatic acid, an alpha or beta hydroxyl acid an organic base or a nitrogen containing compound.

In one or more further embodiments the modulating agent is used to describe an agent, which is a chelating or sequestering or complexing agent that is sufficiently soluble or functional in the solvent to enable it to "mop up" or "lock" metal ions.

In an embodiment modulating agent is used to describe an agent which can effect pH in an aqueous solution. The term modulating agent more particularly means an acid or base or buffer system or combinations thereof, which is introduced into or is present in and acts to modulate the ionic or polar characteristics and any acidity or basesity balance of an emulsion carrier, composition, foamable carrier or foamable composition or resultant foam of the present invention.

In other embodiments modulating agent is used to describe an agent which can effect pH in an aqueous solution. The term modulating agent more particularly means an acid or base or buffer system or combinations thereof, which is introduced into or is present in and acts to modulate the ionic or polar characteristics and any acidity or basesity balance of a waterless or substantially non aqueous carrier, composition, foamable carrier or foamable composition or resultant foam of the present invention.

The substance or residue can be introduced into the formulation from any one or more of the ingredients, some of which themselves may have acidic or basic properties. For example the polymer or solvent may contain basic residues in which case it may be desirable or beneficial to add an acid. Alternatively the surfactant may contain some acid residues in which case the addition of a base may be desirable and beneficial. In some cases more than one ingredient may contain residues which may ameliorate or compound their significance. For example if one ingredient provided weak acid residues and another stronger acid residues the pH in an emulsion environment (or artificial pH in a waterless environment) should be lower. In contrast if one residue was acid and the other basic the net effect in the formulation maybe significantly reduced. In some circumstances the active ingredient may favor an acidic pH or more significantly may need to be maintained at a certain acidic pH otherwise it may readily isomerize, chemically react or breakdown, in which case introducing acidic components such as an acidic polymer might be of help. In an embodiment of the present invention sufficient modulating agent is added to achieve a pH in which the active agent is preferably stable. In another embodiment of the present invention sufficient modulating agent is added to achieve an artificial pH in which the active agent is preferably stable.

The terms pH, pKa, and pKb, buffers and the like are used in classical measurements of an aqueous solution. Such measurements are artificial in a waterless environment. Nevertheless, reference to and description below of such terms are made for convenience and clarity, since such terms are well defined and understood with reference to aqueous solutions and further due to the lack of an appropriate uniform way of describing and identifying the artificial or virtual pH, pK etc in a waterless environment in relation to the present invention. Although predictions of artificial pH can be made using dilution techniques of measurements of waterless formulations diluted in water they are formulation sensitive and specific and have to be carefully calibrated with complex formulas.

Waterless medium can be polar and protic yet it does not conform to classical ionic behavior.

A buffer, as defined by Van Slyke [Van Slyke, J. Biol. Chem. 52, 525 (1922)], is "a substance which by its presence in solution increases the amount of acid or alkali that must be added to cause unit change in pH."

A buffer solution is a solution of a definite pH made up in such a way that this pH alters only gradually with the addition of alkali or acid. Such a solution consists of a solution of a salt of the weak acid in the presence of the three acid itself. The pH of the solution is determined by the dissociation equilibrium of the free acid.

An acid can be a strong acid or a weak acid. A strong acid is an acid, which is a virtually 100% ionized in solution. In contrast, a weak acid is one which does not ionize fully when it is dissolved in water. The lower the value for pKa, the stronger is the acid and likewise, the higher the value for pKa the weaker is the acid.

A base can be a strong base or a weak base. A strong base is something, which is fully ionic with 100% hydroxide ions. In contrast, a weak base is one which does not convert fully into hydroxide ions in solution. The lower the value for pKb, the stronger is the base and likewise, the higher the value for pKb the weaker is the base.

In one or more embodiments of the present invention the modulating agent comprises an organic compound.

In one or more preferred embodiments of the present invention the chelating agent is selected from the group consisting of ethylenediaminetetraacetic acid (EDTA), diethylenetriaminepentaacetic acid (DTPA), hydroxyethylenediaminetriacetic acid (HEDTA), nitrilotriacetic acid (NTA), O,O'-bis(2-aminoethyl)ethyleneglycol-N,N,N',N'-tetraacetic acid (EGTA), trans-1,2-diaminocyclohexane-N,N,N',N'-tetraacetic acid (CyDTA) or a pharmaceutically acceptable salt thereof (normally as a sodium salt), more preferably EDTA, HEDTA and their salts, most preferably EDTA and its salts.

In one or more embodiments of the present invention a preferred non limiting example of the chelating agent is EDTA. Typically, the chelating and sequestering agent is present in the composition at a level of up to about 5.0%, preferably 1.0 percent, by weight, of the composition.

In one or more embodiments of the present invention the modulating agent may also be a preservative or an antioxidant or an ionization agent. Any preservative, antioxidant or ionization agents suitable for pharmaceutical or cosmetic application may be used. Non limiting examples of antioxidants are tocopherol succinate, propyl galate, butylated hydroxy toluene and butyl hydroxy anisol. Ionization agents may be positive or may be negative depending on the environment and the active agent or composition that is to be protected. Ionization agents may for example act to protect or reduce sensitivity of active agents. Non limiting examples of positive ionization agents are benzyl conium chloride, and cetyl pyridium chloride. Non limiting examples of negative ionization agents are sodium lauryl sulphate, sodium lauryl lactylate and phospholipids.

## Humectant

A humectant is a substance that helps retain moisture and also prevents rapid evaporation. Non limiting examples are propylene glycol, propylene glycol derivatives, glycerin, hydrogenated starch hydrosylate, hydrogenated lanolin, lanolin wax, D manitol, sorbitol, sodium 2-pyrrolidone-5-carboxylate, sodium lactate, sodium PCA, soluble collagen, dibutyl phthalate, and gelatin. Other examples may be found in the Handbook of Pharmaceutical Additives published by Gower.

## Moisturizers

A moisturizer, is a substance that helps retain moisture or add back moisture to the skin. Examples are allantoin, petrolatum, urea, lactic acid, sodium PCV, glycerin, shea butter, caprylic/capric/stearic triglyceride, candelilla wax, propylene glycol, lanolin, hydrogenated oils, squalene, sodium hyaluronate and lysine PCA. Other examples may be found in the Handbook of Pharmaceutical Additives published by Gower.

Pharmaceutical compositions of the present invention may in one or more embodiments usefully comprise in addition a heumectant or a moisturizer or combinations thereof.

## Polar solvent

Optionally, the foamable vehicle further includes at least one polar solvent.

A "polar solvent" is an organic solvent, typically soluble in both water and oil. Certain polar solvents, for example propylene glycol and glycerin, possess the beneficial property of a heumectant.

In one or more embodiments, the polar solvent is a heumectant.

In one or more embodiments, the polar solvent is a polyol. Polyols are organic substances that contain at least two hydroxy groups in their molecular structure.

In one or more embodiments, the polar solvent contains an diol (a compound that contains two hydroxy groups in its molecular structure), such as propylene glycol (e.g., 1,2-propylene glycol and 1,3-propylene glycol), butaneediol (e.g., 1,4-butaneediol), butaneediol (e.g., 1,3-butaneediol and 1,4-butenediol), butynediol, pentanediol (e.g., 1,5-pentanediol), hexanediol (e.g., 1,6-hexanediol), octanediol (e.g., 1,8-octanediol), neopentyl glycol, 2-methyl-1,3-propanediol, diethylene glycol, triethylene glycol, tetraethylene glycol, dipropylene glycol and dibutylene glycol.

In one or more embodiments, the polar solvent contains a triol (a compound that contains three hydroxy groups in its molecular structure), such as glycerin and 1,2,6-Hexanetriol.

Other non-limiting examples of polar solvents include pyrrolidones, (such as N-methyl-2-pyrrolidone and 1-methyl-2-pyrrolidinone), dimethyl isosorbide, 1,2,6-hexapetriol, dimethyl sulfoxide (DMSO), ethyl proxitol, dimethylacetamide (DMAc) and alpha hydroxy acids, such as lactic acid and glycolic acid.

According to still other embodiments, the polar solvent is a polyethylene glycol (PEG) or PEG derivative that is liquid at ambient temperature, including PEG200 (MW (molecular weight) about 190-210 kD), PEG300 (MW about 285-315 kD), PEG400 (MW about 380-420 kD), PEG600 (MW about 570-630 kD) and higher MW PEGs such as PEG 4000, PEG 6000 and PEG 10000 and mixtures thereof.

Polar solvents are known to enhance the penetration of active agent into the skin and through the skin, and therefore, their inclusion in the composition of the present invention can be desirable, despite their undesirable skin drying and irritation potential. There is at one level a commonality between the different polar solvents and their penetration enhancement properties. Lower molecular weight alcohols can sometimes be more potent as a solvent, for example by extracting lipids from the skin layers more effectively, which characteristic can adversely affect the skin structure and cause dryness and irritation. Therefore the selection of lower molecular weight alcohols is ideally avoided.

Polar solvents, such as detailed below possess high solubilizing capacity and contribute to the skin penetration of an active agent. Non limiting examples include dimethyl isosorbide, polyols, such as glycerol (glycerin), propylene glycol, hexylene glycol, diethylene glycol, propylene glycol n-alkanols, terpenes, di-terpenes, tri-terpenes, limonene, terpene-ol, 1-menthol, dioxolane, ethylene glycol, other glycols, oleyl alcohol, alpha-hydroxy acids, such as lactic acid and glycolic acid, sulfoxides, such as dimethylsulfoxide (DMSO), dimethylformanide, methyl dodecyl sulfoxide, dimethylacetamide, azone (1-dodecylazacycloheptan-2-one), 2-(n-nonyl)-1,3-dioxolane, alkanols, such as dialkylamino acetates, and admixtures thereof. In certain preferred embodiments, the polar solvent is selected from the group consisting of dimethyl isosorbide glycerol (glycerin), propylene glycol, hexylene glycol, terpene-ol, oleyl alcohol, lactic acid and glycolic acid.

**Skin penetration enhancer**

A "skin penetration enhancer", also termed herein "penetration enhancer," is an organic solvent, typically soluble in both water and oil. Examples of penetration enhancer include polyols, such as glycerol (glycerin), propylene glycol, hexylene glycol, diethylene glycol, propylene glycol n-alkanols, terpenes, di-terpenes, tri-terpenes, terpenols, limonene, terpene-ol, 1-menthol, dioxolane, ethylene glycol, hexylene glycol, other glycols, sulfoxides, such as dimethylsulfoxide (DMSO), dimethylformanide, methyl dodecyl sulfoxide, dimethylacetamide, dimethylisosorbide, monooleate of ethoxylated glycerides (with 8 to 10 ethylene oxide units), azone (1-dodecylazacycloheptan-2-one), 2-(n-nonyl)-1,3-dioxolane, esters, such as isopropyl myristate/palmitate, ethyl acetate, butyl acetate, methyl proprionate, capric/caprylic triglycerides, octylmyristate, dodecyl-myristate; myristyl alcohol, lauryl alcohol, lauric acid, lauryl lactate ketones; amides, such as acetamide oleates such as triolein; various alkanoic acids such as caprylic acid; lactam compounds, such as azone; alkanols, such as dialkylamino acetates, and admixtures thereof.

According to one or more embodiments, the penetration enhancer is a polyethylene glycol (PEG) or PEG derivative that is liquid at ambient temperature

**Potent Solvent**

In one or more embodiments of the present invention, the foamable composition includes a potent solvent, in addition to or in place of one of the hydrophobic solvents, polar solvents or emollients of the composition. A potent solvent is a solvent other than mineral oil that solubilizes a specific active agent substantially better than a hydrocarbon solvent such as mineral oil or petrolatum. For example, a potent solvent solubilizes the active agent 5 fold better than a hydrocarbon solvent; or even solubilizes the active agent 10-fold better than a hydrocarbon solvent.

In one or more embodiments of the present invention, the composition includes at least one active agent in a therapeutically effective concentration; and at least one potent solvent in a sufficient amount to substantially solubilize the at least one active agent in the composition. The term "substantially soluble" means that at least 95% of the active agent has been solubilized, i.e., 5% or less of the active agent is present in a solid state. In one or more embodiments, the concentration of the at least one potent solvent is more than about 40% of the at least one solvent of the composition of the present invention; or even more than about 60%.

Non-limiting examples of pairs of active agent and potent solvent include: Betamethasone valerate: Practically insoluble in mineral oil (<0.01%); soluble more than 1% in glycofurol; Hydrocortisone butyrate: Practically insoluble in mineral oil (<0.01%); soluble more than 1% in glycofurol; Metronidazole: Practically insoluble in mineral oil (<0.01%); soluble more than 1% in dimethyl isosrbide; Ketoconazole: Practically insoluble in mineral oil (<0.01%); soluble more than 1% in glycofurol, propylene glycol and dimethyl isosrbide; Mupirocin: Practically insoluble in mineral oil (<0.01%); soluble more than 1% in glycofurol, hexylene glycol, dimethyl isosorbide, propylene glycol and polyethylene glycol 400 (PEG 400); Meloxicam, a nonsteroidal anti-inflammatory agent: Practically insoluble in mineral oil (<0.001%); soluble in propylene glycol: 0.3 mg/mL; and in PEG 400: 3.7 mg/mL; and Progesterone: Practically insoluble in mineral oil (<0.001%); soluble in PEG 400: 15.3 mg/mL.

A non-limiting exemplary list of solvents that can be considered as potent solvents includes polyethylene glycol, propylene glycol, hexylene glycol, butaneediols and isomers thereof, glycerol, benzyl alcohol, DMSO, ethyl oleate, ethyl caprylate, diisopropyl adipate, dimethylacetamide, N-methylpyrrolidone, N-hydroxyethylpyrrolidone, polyvinylpyrrolidone, isosorbide derivatives, such as dimethyl isosorbide, glycofurol and ethoxydiglycol (transcutol) and laurocapram .

The use of a potent solvent in a foam composition provides an improved method of delivering poorly soluble therapeutic agents to a target area. It is known that low drug solubility results in poor bioavailability, leading to decreased effectiveness of treatment. Foam compositions of the present invention, for which the solvent includes a potent solvent, increase the levels of the active agent in solution and thus, provide high delivery and improved therapy.

Potent solvents, as defined herein, are usually liquid. Formulations comprising potent solvents and active agents are generally disadvantageous as therapeutics, since their usage involves unwanted dripping and inconvenient method of application; resulting in inadequate dosing. Surprisingly, the foams of the present invention, which are drip-free, provide a superior vehicle for such active agents, enabling convenient usage and accurate effective dosing.

In one or more embodiments of the present invention the present invention the foamable pharmaceutical composition may additionally include a mixture of two or more of the solvents selected from the group of hydrophobic solvents, silicone oils, emollients, polar solvents and potent solvents in an appropriate proportion as would be appreciated to a person skilled in the art.

In one or more embodiments of the present invention, the PPG alkyl ether may act as a potent solvent

**Additional components**

In an embodiment of the present invention, a composition of the present invention includes one or more additional components. Such additional components include but are not limited to anti perspirants, anti-static agents, buffering agents, bulking agents, chelating agents, cleansers, colorants, conditioners, deodorants, diluents, dyes, emollients, fragrances, hair conditioners, humectants, pearlescent aids, perfuming agents, permeation enhancers, pH-adjusting agents, preservatives, protectants, skin penetration enhancers, softeners, solubilizers, sunscreens, sun blocking agents, sunless tanning agents, viscosity modifiers and vitamins. As is known to one skilled in the art, in some instances a specific additional component may have more than one activity, function or effect.

**Propellants**

Suitable propellants include volatile hydrocarbons such as butane, propane, isobutane and fluorocarbon gases, or mixtures thereof.

The propellant makes up about 5-25 wt% of the foamable composition. The propellants are used to generate and administer the foamable composition as a foam. The total composition including propellant, foamable compositions and optional ingredients is referred to as the foamable composition.

Alcohol and organic solvents render foams inflammable. It has been surprisingly discovered that fluorohydrocarbon propellants, other than chloro-fluoro carbons (CMCs), which are non-ozone-depleting propellants, are particularly useful in the production of a non-flammable foamable composition. A test according to European Standard prEN 14851, titled "Aerosol containers - Aerosol foam flammability test" revealed that compositions containing an organic carrier that contains a hydrophobic organic carrier and/or a polar solvent, which are detected as inflammable when a hydrocarbon propellant is used, become non-flammable, while the propellant is an HFC propellant.

Such propellants include, but are not limited to, hydrofluorocarbon (HFC) propellants, which contain no chlorine atoms, and as such, fall completely outside concerns about stratospheric ozone destruction by chlorofluorocarbons or other chlorinated hydrocarbons. Exemplary non-flammable propellants according to this aspect of the invention include propellants made by DuPont under the registered trademark Dymel, such as 1,1,1,2 tetrafluorethane (Dymel 134), and 1,1,1,2,3,3,3 heptafluoropropane (Dymel 227). HFCs possess Ozone Depletion Potential of 0.00 and thus, they are allowed for use as propellant in aerosol products.

Notably, the stability of foamable emulsions including HFC as the propellant can be improved in comparison with the same composition made with a hydrocarbon propellant.

In one or more embodiments foamable compositions comprise a combination of a HFC and a hydrocarbon propellant such as n-butane or mixtures of hydrocarbon propellants such as propane, isobutane and butane.

**MICROEMULSIONS AND NANOEMULSIONS**

Microemulsions and nanoemulsion are monophasic, transparent (or slightly translucent) dispersions of oil and water. Unlike conventional emulsions, microemulsions and nanoemulsion are thermodynamically stable, making them a favorable vehicle for pharmaceutical compositions, which have to maintain stability for long periods of time. They and a method of manufacture are more particularly described in US2006/0233721 which is incorporated herein by way of reference. As will be appreciated by a man of the art the methodology may be adapted according to the type of carrier composition.

**AGING**

In order to project the potential shelf life and stability of the compositions and their ingredients particularly active or benefit agents the compositions can be subjected to a number of tests, including centrifugation to look for resistance to creaming, phase separation; one or more freeze thaw cycles, standing at room and higher temperatures as an indicator of resistance to aging.

**Composition and Foam Physical Characteristics and Advantages**

A pharmaceutical or cosmetic composition manufactured using the foamable carrier of the present invention is very easy to use. When applied onto the afflicted body surface of mammals, i.e., humans or animals, it is in a foam state, allowing free application without spillage. Upon further application of a mechanical force, e.g., by rubbing the composition onto the body surface, it freely spreads on the surface and is rapidly absorbed.

The foamable composition of the present invention is stable, having an acceptable shelf-life of at least one year, or preferably, at least two years at ambient temperature, as revealed in accelerated stability tests. The foamable compositions according to the present invention are stable. Following accelerated stability studies, they demonstrate desirable texture; they form fine bubble structures that do not break immediately upon contact with a surface, spread easily on the treated area and absorb quickly.

The composition should also be free flowing, to allow it to flow through the aperture of the container, e.g., and aerosol container, and create an acceptable foam.

Foam quality can be graded as follows:

Grade E (excellent): very rich and creamy in appearance, does not show any bubble structure or shows a very fine (small) bubble structure; does not rapidly become dull; upon spreading on the skin, the foam retains the creaminess property and does not appear watery.

Grade G (good): rich and creamy in appearance, very small bubble size, "dulls" more rapidly than an excellent foam, retains creaminess upon spreading on the skin, and does not become watery.

Grade FG (fairly good): a moderate amount of creaminess noticeable, bubble structure is noticeable; upon spreading on the skin the product dulls rapidly and becomes somewhat lower in apparent viscosity.

Grade F (fair): very little creaminess noticeable, larger bubble structure than a "fairly good" foam, upon spreading on the skin it becomes thin in appearance and watery.

Grade P (poor): no creaminess noticeable, large bubble structure, and when spread on the skin it becomes very thin and watery in appearance.

Grade VP (very poor): dry foam, large very dull bubbles, difficult to spread on the skin.

Topically administrable foams are typically of quality grade E or G, when released from the aerosol container. Smaller bubbles are indicative of more stable foam, which does not collapse spontaneously immediately upon discharge from the container. The finer foam structure looks and feels smoother, thus increasing its usability and appeal.

A further aspect of the foam is breakability. The breakable foam is thermally stable, yet breaks under shear force. Shear-force breakability of the foam is clearly advantageous over thermally induced breakability. Thermally sensitive foams immediately collapse upon exposure to skin temperature and, therefore, cannot be applied on the hand and afterwards delivered to the afflicted area.

Another property of the foam is specific gravity, as measured upon release from the aerosol can. Typically, foams have specific gravity of less than 0.12 g/mL; or less than 0.10 g/mL; or less than 0.08 g/mL, depending on their composition and on the propellant concentration.

**Pharmaceutical composition**

The foamable carrier of the present invention is an ideal vehicle for active pharmaceutical ingredients and active cosmetic ingredients. In the context of the present invention, active pharmaceutical ingredients and active cosmetic ingredients are collectively termed "active agent" or "active agents."

In one or more embodiments, the azelaic acid is the active ingredient. It can be used in the formulation as a suspended solid or in solution, alone or in combination with other active agents. As is known to one skilled in the art, in some instances a specific active agent may have more than one activity, function or effect.

In one embodiment, the azelaic acid is useful as an antibiotic, an antifungal agent, a keratolytic agent, an inhibitor of the reduction of testosterone to dihydrotestosterone, an inhibitor of the production of sebum in the sebaceous gland, an anti-acne agent, by way of example. Dicarboxylic acids, and azelaic acid in particular, may be used for the treatment of diaper rash, hyperpigmentary drmatoses, acne, presbyderma of aging skin, hyperhydrosis, ischthyosis, and wrinkling of the skin, antitumor agents (for example, in conjunction with vitamins A, E and D), rosacea, a pigmentation disorder, a cell proliferation abnormality a skin infection and a skin inflammation and treatment of corns and callouses due to the anti-keratolytic effects.

In one or more embodiments, the dicarboxylic acid or dicarboxylic ester is used as a solvent for an active agent or as a penetration enhancer for an active agent.

Suitable active agents for use in conjunction with a dicarboxylic acid or a dicarboxylic ester include, but are not limited to, active herbal extracts, acaricides, age spot and keratose removing agents, allergen, analgesics, local anesthetics, antiacne agents, antiallergic agents, antiaging agents, antibacterials, antibiotics, antiburn agents, anticancer agents, antidandruff agents, antidepressants, antidermatitis agents, antiedemics, antihistamines, antihelminths, antihyperkeratolyte agents, antiinflammatory agents, antiirritants, antilipemics, antimicrobials, antimycotics, antiproliferative agents, antioxidants, anti-wrinkle agents, antipruritics, antipsoriatic agents, antirosacea agents antiseborrheic agents, antiseptic, antiswelling agents, antiviral agents, antiyeast agents, astringents, topical cardiovascular agents, chemotherapeutic agents, corticosteroids, dicarboxylic acids, disinfectants, fungicides, hair growth regulators, hormones, hydroxy acids, immunosuppressants, immunoregulating agents, insecticides, insect repellents, keratolytic agents, lactams, metals, metal oxides, mitocides, neuropeptides, non-steroidal anti-inflammatory agents, oxidizing agents, pediculicides, photodynamic therapy agents, retinoids, sanatives, scabicides, self tanning agents, skin whitening agents, vasoconstrictors, vasodilators, vitamins, vitamin D derivatives, wound healing agents and wart removers. As is known to one skilled in the art, in some instances a specific active agent may have more than one activity, function or effect.

In one or more embodiments, the formulation may additionally include a steroidal anti-inflammatory agent. A dicarboxylic acid ester may be present in the composition in an amount sufficient to solubilize the steroid. Exemplary steroidal anti-inflammatory agents include, but are not limited to, corticosteroids such as bydrocortisone, hydroxyltriamcinolone, alpha-methyl dexamethasone, dexamethasone-phosphate, beclomethsone dipropionate, clobetasol valemate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylester, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylpropionate, hydrocortmate, mepreddisone, paramethasone, prednisolone, prednisone, beclomethasone dipropionate, triamcinolone, and mixtures thereof. In an embodiment of the present invention, the dicarboxylic acid ester is present in the composition in an amount sufficient to solubilize the steroid.

In one embodiment, the formulation may additionally include an immunomodulator. A dicarboxylic acid ester may be present in the composition in an amount sufficient to solubilize the immunomodulator. Immunomodulators are chemically or biologically-derived agents that modify the immune response or the functioning of the immune system (as by the stimulation of antibody formation or the inhibition of white blood cell activity). Immunomodulators include, among other options, cyclic peptides, such as cyclosporine, tacrolimus, tresperimus, pimecrolimus, sirolimus (rapamycin), verolimus, laflunimus, laquinimod and imiquimod. Such compounds, delivered in the foam of the present invention, are especially advantageous in skin disorders such as psoriasis, eczema and atopic dermatitis, where the large skin areas are to be treated.

In an embodiment of the present invention, the active agent is selected from a dicarboxylic acid and a dicarboxylic acid ester.

Because of the multiple therapeutic properties of dicarboxylic acids and their respective esters, the combination of such dicarboxylic acids or their respective esters with another active agents can result in a synergistic therapeutic benefit. For example, psoriasis is characterized by a heperkeratinization aspect and an inflammation, and therefore, its treatment can benefit from the combination of a dicarboxylic acid, which is keratolytic and a steroid.

**Fields of Applications**

The foamable carrier of the present invention is suitable for treating any inflicted surface. In one or more embodiments, foamable carrier is suitable for administration to the skin, a body surface, a body cavity or mucosal surface, e.g., the cavity and/or the mucosa of the nose, mouth, eye, ear, respiratory system, vagina or rectum (severally and interchangeably termed herein "target site").

In one embodiment, the disorder is a dermatological disorder, which can be treated by a dicarboxylic acid.

In another embodiment, the disorder is a dermatological disorder that can benefit from the use of a dicarboxylic acid or dicarboxylic ester in conjunction with another active agent. The dicarboxylic acid or dicarboxylic ester may benefit by improving the solubility of the active agent or increasing the penetration of the active agent. The dicarboxylic acid or dicarboxylic ester may also provide a synergistic therapeutic effect in combination with the active agent.

By selecting a suitable active agent, or a combination of two or more active agents, the foamable composition of the present invention is useful in treating an animal or a human patient having any one of a variety of dermatological disorders, including dermatological pain, dermatological inflammation, acne, acne vulgaris, inflammatory acne, non-inflammatory acne, acne fulminans, nodular papulopustular acne, acne conglobata, dermatitis, bacterial skin infections, fungal skin infections, viral skin infections, parasitic skin infections, skin neoplasia, skin neoplasms, pruritis, cellulitis, acute lymphangitis, lymphadenitis, erysipelas, cutaneous abscesses, necrotizing subcutaneous infections, scalded skin syndrome, folliculitis, furuncles, hidradenitis suppurativa, carbuncles, paronychial infections, rashes, erythrasma, impetigo, ecthyma, yeast skin infections, warts, molluscum contagiosum, trauma or injury to the skin, post-operative or post-surgical skin conditions, scabies, pediculosis, creeping eruption, eczemas, psoriasis, pityriasis rosea, lichen planus, pityriasis rubra pilaris, edematous, erythema multiforme, erythema nodosum, granuloma annulare, epidermal necrolysis, sunburn, photosensitivity, pemphigus, bullous pemphigoid, dermatitis herpetiformis, keratosis pilaris, callouses, corns, ichthyosis, skin ulcers, ischemic necrosis, miliaria, hyperhidrosis, moles, Kaposi's sarcoma, melanoma, malignant melanoma, basal cell carcinoma, squamous cell carcinoma, poison ivy, poison oak, contact dermatitis, atopic dermatitis, rosacea, purpura, moniliasis, candidiasis, baldness, alopecia, Behcet's syndrome, cholesteatoma, Dercum disease, ectodermal dysplasia, gustatory sweating, nail patella syndrome, lupus, hives, hair loss, Hailey-Hailey disease, chemical or thermal skin burns, scleroderma, aging skin, wrinkles, sun spots, necrotizing fasciitis, necrotizing myositis, gangrene, scarring, and vitiligo.

Likewise, the foamable composition of the present invention is suitable for treating a disorder of a body cavity or mucosal surface, e.g., the mucosa of the nose, mouth, eye, ear, respiratory system, vagina or rectum. Non limiting examples of such conditions include chlamydia infection, gonorrhea infection, hepatitis B, herpes, HIV/AIDS, human papillomavirus (HPV), genital warts, bacterial vaginosis, candidiasis, chancroid, granuloma Inguinale, lymphogranuloma venereum, mucopurulent cervicitis (MPC), molluscum contagiosum, nongonococcal urethritis (NGU), trichomoniasis, vulvar disorders, vulvodynia, vulvar pain, yeast infection, vulvar dystrophy, vulvar intraepithelial neoplasia (VIN), contact dermatitis, pelvic inflammation, endometritis, salpingitis, oophoritis, genital cancer, cancer of the cervix, cancer of the vulva, cancer of the vagina, vaginal dryness, dyspareunia, anal and rectal disease, anal abscess/fistula, anal cancer, anal fissure, anal warts, Crohn's disease, hemorrhoids, anal itch, pruritus ani, fecal incontinence, constipation, polyps of the colon and rectum.

In an embodiment of the present invention, the disorder is a dermatological disorder, which can be treated by a dicarboxylic acid.

In an embodiment of the present invention, the disorder is a dermatological disorder, which can be treated by a dicarboxylic acid ester.

In an embodiment of the present invention, the disorder is a dermatological disorder, which can be treated by a topical steroid, and the dicarboxylic acid or dicarboxylic ester provides a beneficial effect by increasing the solubility or penetration of the topical steroid.

In an embodiment of the present invention, the disorder is a dermatological disorder, which can be treated by an immunomodulator and the dicarboxylic acid or dicarboxylic ester provides a beneficial effect by increasing the solubility or penetration of the topical immunomodulator.

In an embodiment of the present invention, the disorder is a dermatological disorder, which can be treated by an anti-infective agent, such as an antibacterial agent, and antibiotic, an antifungal agent and an antiviral agent, and the dicarboxylic acid or dicarboxylic ester provides a beneficial effect as an anti-infective agent or by increasing the solubility or penetration of the anti-infective agent.

In an embodiment of the present invention, the disorder is a dermatological disorder, which is common in children. Foam is advantageous in the topical treatment of children, who are sensitive to treatment with a cream or ointment.

In an embodiment of the present invention, the disorder is atopic dermatitis and the active agent is a steroid, further including a dicarboxylic acid (DCA) or DCA ester to stabilize or solubilize the topical steroid.

In an embodiment of the present invention, the disorder is psoriasis and the active agent is a steroid, further including a DCA or DCA ester to stabilize or solubilize the topical steroid.

In an embodiment of the present invention, the disorder is selected from psoriasis and atopic dermatitis and the active agent comprises a steroid and an additional non-steroidal active agent, such as a vitamin D derivative, further including a DCA or DCA ester to stabilize or solubilize the topical steroid and/or non-steroidal active agent.

In an embodiment of the present invention, the disorder is selected from psoriasis and atopic dermatitis and the active agent comprises an immunomodulator, further including a DCA or DCA ester to stabilize or solubilize the immunomodulator.

In an embodiment of the present invention, the composition is useful for the treatment of an infection. In one or more embodiments, the composition is suitable for the treatment of an infection, selected from the group of a bacterial infection, a fungal infection, a yeast infection, a viral infection and a parasitic infection.

In an embodiment of the present invention, the composition is useful for the treatment of wound, ulcer and burn.

The composition of the present invention is also suitable for administering a hormone to the skin or to a mucosal membrane or to a body cavity, in order to deliver the hormone into the tissue of the target organ, in any disorder that responds to treatment with a hormone.

Other foamable compositions are described in: U.S. Publication No. 05-0232869, published on October 20, 2005, entitled NONSTEROIDAL IMMUNOMODULATING KIT AND COMPOSITION AND USES THEREOF; U.S. Publication No. 05-0205086, published on September 22, 2005, entitled RETINOID IMMUNOMODULATING KIT AND COMPOSITION AND USES THEREOF; U.S. Publication No. 06-0018937, published on January 26, 2006, entitled STEROID KIT AND FOAMABLE COMPOSITION AND USES THEREOF; U.S. Publication No. 05-0271596, published on December 8, 2005, entitled VASOACTIVE KIT AND COMPOSITION AND USES THEREOF; U.S. Publication No. 06-0269485, published on November 30, 2006, entitled ANTIBIOTIC KIT AND COMPOSITION AND USES THEREOF; U.S. Publication No. 07-0020304, published on January 25, 2007, entitled NON-FLAMMABLE INSECTICIDE COMPOSITION AND USES THEREOF; U.S. Publication No. 06-0193789, published on August 31, 2006, entitled FILM FORMING FOAMABLE COMPOSITION; U.S. Patent Application No. 11/732547, filed on April 4, 2007, entitled ANTI-INFECTION AUGMENTATION OF FOAMABLE COMPOSITIONS AND KIT AND USES THEREOF; U.S. Provisional Patent Application No. 60/789186, filed on April 4, 2006, KERATOLYTIC ANTIFUNGAL FOAM; U.S. Provisional Patent Application No. 0/815948, filed on June 23, 2006, entitled FOAMABLE COMPOSITIONS COMPRISING A CALCIUM CHANNEL BLOCKER, A CHOLINERGIC AGENT AND A NITRIC OXIDE DONOR; U.S. Provisional Patent Application No. 60/818634, filed on July 5, 2006, entitled DICARBOXYLIC ACID FOAMABLE VEHICLE AND PHARMACEUTICAL COMPOSITIONS THEREOF; U.S. Provisional Patent Application No. 60/843140, filed on September 8, 2006, entitled FOAMABLE VEHICLE AND VITAMIN PHARMACEUTICAL COMPOSITIONS THEREOF, all of which are incorporated herein by reference in their entirety. More particularly any of the active ingredients; the solvents; the surfactants; foam adjuvants; penetration enhancers; humectants; moisturizers; and other excipients as well as the propellants listed therein can be applied herein and are incorporated by reference.

The following examples further exemplify the azelaic acidfoamable pharmaceutical carriers, pharmaceutical compositions thereof, methods for preparing the same, and therapeutic uses of the compositions. The examples are for the purposes of illustration only and are not intended to be limiting of the invention. Many variations may be carried out by one of ordinary skill in the art and are contemplated within the full scope of the present invention.

## METHODOLOGY

A general procedure for preparing foamable compositions is set out in WO 2004/037225, which is incorporated herein by reference.

### Emulsion Foam

    1. Mix oily phase ingredients and heat to 75°C to melt all ingredients and obtain homogeneous mixture.
    2. Mix polymers in water with heating or cooling as appropriate for specific polymer.
    3. Add all other water soluble ingredients to water-polymer solution and heat to 75°C.
    4. Add slowly internal phase to external phase at 75°C under vigorous mixing and homogenize to obtain fine emulsion. Alternatively the external phase is added slowly to the internal phase.
    5. Cool to below 40°C and add sensitive ingredients with mild mixing.
    6. Cool to room temperature.

### Waterless Foam

    1. Dissolve the polymers in the main solvent with heating or cooling as appropriate for specific polymer. Add the all other ingredients and heat to 75°C to melt and dissolve the various ingredients.
    2. Cool to below 40°C and add sensitive ingredients with mild mixing.
    3. Cool to room temperature.

**Oily Waterless Foam**

1. Mix all ingredients excluding polymers and heat to 75°C to melt and dissolve and obtain homogeneous mixture.
2. Mix well and cool to below 40°C and add the polymers and sensitive ingredients with moderate mixing.
3. Cool to room temperature.

**Oily Foam with phospholipids and/or water**

1. Swell the phospholipids in the main oily solvent under mixing for at least 20 minutes until uniform suspension is obtained.
2. Add all other ingredients excluding polymers and heat to 75°C to melt and dissolve and obtain homogeneous mixture.
3. Mix well and cool to below 40°C and add the polymers and sensitive ingredients with moderate mixing.
4. Cool to room temperature.
5. In case of polymers dissolved in water or organic solvent, dissolve the polymers in the solvent with heating or cooling as appropriate for specific polymer and add to the oily mixture under vigorous mixing at ~40°C.

Canisters Filling and Crimping

Each aerosol canister is filled with PFF and crimped with valve using vacuum crimping machine.

Pressurizing

Propellant Filling

Pressurizing is carried out using a hydrocarbon gas or gas mixture Canisters are filled and then warmed for 30 sec in a warm bath at 50°C and well shaken immediately thereafter.

Closure Integrity Test.

Each pressurized canister is subjected to bubble and crimping integrity testing by immersing the canister in a 60°C water bath for 2 minutes. Canisters are observed for leakage as determined by the generation of bubbles. Canisters releasing bubbles are rejected.

**TESTS**

By way of non limiting example the objectives of hardness, collapse time and FTC stability tests are briefly set out below as would be appreciated by a person of the art.

Hardness

LFRA100 instrument is used to characterize hardness. A probe is inserted into the test material. The resistance of the material to compression is measured by a calibrated load cell and reported in units of grams on the texture analyzer instrument display. Preferably at least three repeat tests are made. The textural characteristics of a dispensed foam can effect the degree of dermal penetration, efficacy, spreadability and acceptability to the user. The results can also be looked at as an indicator of softness. Note: the foam sample is dispensed into an aluminum sample holder and filled to the top of the holder.

Collapse Time

Collapse time (CT) is examined by dispensing a given quantity of foam and photographing sequentially its appearance with time during incubation at 36°C. It is useful for evaluating foam products, which maintain structural stability at skin temperature for at least 1 min.

Viscosity

Viscosity is measured with Brookfield LVDV-II + PRO with spindle SC4-25 at ambient temperature and 10, 5 and 1 RPM. Viscosity is usually measured at 10RPM. However, at about the apparent upper limit for the spindle of ~>50,000CP, the viscosity at 1 RPM may be measured, although the figures are of a higher magnitude.

FTC (Freeze Thaw Cycles)

To check the foam appearance under extreme conditions of repeated cycles of cooling, heating, (first cycle) cooling, heating (second cycle) etc., commencing with -100°C (24hours) followed by +400°C (24hours) measuring the appearance and again repeating the cycle for up to three times.

Creaming by centrifugation:

1. Principle of test
The centrifugation used in this procedure serves as a stress condition simulating the aging of the liquid dispersion under investigation. Under these conditions, the centrifugal force applied facilitates the coalescence of dispersed globules or sedimentation of dispersed solids, resulting in loss of the desired properties of the formulated dispersion.
2. Procedure

2.1. Following preparation of the experimental formulation/s, allow to stand at room temperature for $\geq$ 24 h.
2.2. Handle pentane in the chemical hood. Add to each experimental formulation in a 20-mL glass vial a quantity of pentane equivalent to the specified quantity of propellant for that formulation, mix and allow formulation to stand for at least 1 h and not more than 24 h.
2.3. Transfer each mixture to 1.5 mL microtubes. Tap each microtube on the table surface to remove entrapped air bubbles.
2.4. Place visually balanced microtubes in the centrifuge rotor and operate the centrifuge at 3,000 rpm for 10 min or at 1,000 rpm for 10 min.

Intra-canister uniformity

1. Representative product containers are collected, sample test solutions are prepared and the content of the analyte is determined according to standard methods in the art. Variability of content is characterized as percent difference or relative standard deviation, as appropriate, according to the number of samples evaluated.
2. The results ascertain variability or uniformity within a given container in content of analytes (primarily active pharmaceutical ingredients, but also preservatives) taken from different parts of a pressurized canister drug products.
3. Two full canisters were shaken according to product instructions. About 1-3 g of Foam was dispensed from each canister and discarded. Foam sufficient for two replicate sample solution preparations was then dispensed into a glass beaker. This represents the initial sample. A middle portion is then dispensed from each canister being about half the canister contents. This middle dispensed portion may be discarded or collected for testing purposes, as necessary. Foam sufficient for two replicate sample solution preparations was then dispensed into a glass beaker. This represents the final sample. A small amount of formulation remains in the canister. The foam samples were stirred to remove gas / air bubbles. From both the initial and final foam portions from each canister 4 separate sample solutions are prepared and analyzed, 2 from the initial portion and 2 from the final portion. The percent difference is calculated as follows:

$$\frac{\text{Difference between content determined in initial \& final portions}}{\text{Mean of content of initial \& final portions}} \times 100$$

and the intra canister uniformity evaluated from the results.

**STOCK COMPOSITIONS**

Non-limiting examples of how stock solutions are made up with and without API. Other stock solutions may be made using the same methodology by simply varying adding or omitting ingredients as would be appreciated by one of the ordinary skills in the art.

**Examples**

The invention is described with reference to the following examples. This invention is not limited to these examples and experiments. Many variations will suggest themselves and are within the full intended scope of the claims.

**SUSPENSIONS**

Example 1 - Dicarboxylic acid composition

The following foamable vehicles were prepared and the quality of the resultant foam was ascertained.

Part A -Formulation

| Ingredient | %w/w |
| --- | --- |
| Azelaic Acid | 15.00 |
| Water | 51.90 |
| Caprylic/Capric triglyceride | 10.87 |
| Propylene glycol | 10.87 |
| Dimethyl isosorbide | 5.44 |
| PEG-40 stearate | 2.83 |
| Cetostearyl alcohol | 1.09 |
| Polysorbate 80 | 0.98 |
| Glyceryl stearate | 0.54 |
| Xanthan gum | 0.27 |
| Methylcellulose A4M | 0.11 |
| Benzoic acid | 0.10 |
| NaOH (18% Solution) | to pH=4.5 |
| Total: | 100 |

Notes

- The composition contains azelaic acid as a benefit agent, which is suitable for treating a skin disorder, selected from acne, rosacea, a pigmentation disorder, a cell proliferation abnormality a skin infection and a skin inflammation.
- The composition contains about 10% capric/caprylic triglyceride to provide emolliency and about 10% propylene glycol and 10% dimethyl isosorbide, to provide (1) enhanced emolliency; (2) improved solubilizing capacity of the azelaic acid; and (3) enhanced skin delivery.
- The compositions contain about 50% water. Therefore, they provide high skin barrier build-up effect.
- The composition can be used as a cream/lotion for topical therapy of a skin diorder.
- In order to create a foamable composition, the composition is filled into an aerosol canister and pressurized using a liquefied or gas propellant can be added at a concentration of about 3% to about 25%.

Part B - 12 month stability test

| Test parameter | | T-0 | T-12 | |
|---|---|---|---|---|
| | | | upright | inverted |
| API* assay by HPLC (%) | % amount of label | 99.3 | 100.5 | 101.7 |
| | % w/w | 14.90 | 15.07 | 15.26 |
| Product content uniformity (Intra canister) of API (%) | | 1.79 | 0.10 | 0.88 |
| *API = active pharmaceutical ingredient | | | | |

The total amount of active agent at T-0 and at T-12 months as a percentage of 100% of ingredient that should be present according to the label and as a percentage in the formulation w/w, respectively was determined. As can be seen, no reduction in API content was observed within the limits of detection and that the content remained uniform. The differences between samples taken from the top of the canisters and from samples taken from the bottom of the canisters were not significant and were well within the acceptable range.

Furthermore, the formulation comprising active ingredient azaleic acid - despite being a suspension and subject to graviational effect - was able to withstand sedimentation and degredation such that it has remained stable and uniformly distributed in the formulation as a suspension over a prolonged period of 12 months, whilst remaining flowable and shakable.

Example 2 - Additional dicarboxylic acid compositions

The following foamable vehicles were prepared and the quality of the resultant foam was ascertained.

| Formulation : | AZL018 | AZL03 4 | AZL03 5 | AZL03 6 | AZL03 7 | AZL03 8 | AZL03 9 |
|---|---|---|---|---|---|---|---|
| | %w/w | %w/w | %w/w | %w/w | %w/w | %w/w | %w/w |
| Azelaic Acid | 15.00 | 15.00 | 15.00 | 15.00 | 15.00 | 15.00 | 15.00 |
| Caprylic/capric triglyceride | 5.00 | 10.00 | 5.00 | 5.00 | 5.00 | 5.00 | 10.00 |
| Cetostearyl alcohol | 0.90 | 1.00 | 1.00 | 1.00 | 1.00 | 1.00 | 1.00 |
| Glyceryl stearate | 0.45 | 0.50 | 0.50 | 0.50 | 0.50 | 0.50 | 0.50 |
| Cholesterol | 1.00 | --- | --- | --- | --- | 1.00 | --- |
| Benzoic acid | | 0.20 | --- | 0.20 | 0.20 | 0.20 | --- |
| Benzyl alcohol | 1.00 | --- | 1.00 | --- | --- | --- | 1.00 |
| PEG-40 stearate | | 2.60 | 2.60 | 2.60 | 2.60 | 2.60 | 2.60 |
| Methylcellulose | | 0.10 | 0.10 | 0.10 | 0.10 | 0.10 | 0.10 |
| Hydroxypropyl methylcellulose | 0.10 | --- | --- | --- | --- | --- | --- |
| Xanthan gum | 0.25 | 0.10 | 0.25 | 0.10 | 0.25 | 0.10 | 0.10 |
| Polysorbate 80 | 0.90 | 0.90 | 0.90 | 0.90 | 0.90 | 0.90 | 0.90 |
| EDTA disodium dehydrate | 0.10 | --- | --- | --- | --- | --- | --- |
| PEG-400 | --- | --- | --- | --- | 5.00 | --- | --- |
| Dimethyl isosorbide | 10.00 | --- | --- | 5.00 | --- | --- | 5.00 |
| 50% phosphotidylcholine in propylene glycol | --- | 2.80 | 2.80 | --- | 2.80 | --- | 2.80 |

(continued)

| Formulation : | AZL018 | AZL03 4 | AZL03 5 | AZL03 6 | AZL03 7 | AZL03 8 | AZL03 9 |
|---|---|---|---|---|---|---|---|
| | %w/w | %w/w | %w/w | %w/w | %w/w | %w/w | %w/w |
| propylene glycol | 6.00 | 5.00 | 10.00 | 10.00 | --- | --- | --- |
| Sodium hydroxide | to pH=4.5 | to pH=4.5 | to pH=5.3 | to pH=4.5 | to pH=4.5 | to pH=4.5 | to pH=5.3 |
| Propellant (butane+ isobutane + propane) | 8.00 | 8.00 | 8.00 | 8.00 | 8.00 | 8.00 | 8.00 |
| Purified water | to 100 | to 100 | to 100 | to 100 | to 100 | to 100 | to 100 |

Example 3 -Compositions with azalaic acid with and without different polymeric agents

The following foamable vehicles were prepared and the quality of the resultant foam was ascertained.

| | DCA021 | DCA023 | DCA023A | DCA026 | DCA026a |
|---|---|---|---|---|---|
| Isopropyl myristate | | | | 11.00 | 11.00 |
| MCT oil | | 35.00 | 35.00 | | |
| Azelaic acid | 20.00 | 15.00 | 15.00 | 15.00 | 15.00 |
| Propylene glycol | 30.00 | | | | |
| Steareth 2 | | 8.00 | 8.00 | | |
| Steareth 21 | | 2.00 | 2.00 | | |
| PEG-40 Stearate | | | | 4.00 | 6.00 |
| Polysorbate 80 | 30.00 | | | 1.40 | 2.10 |
| Xanthan gum | 0.30 | | | 0.27 | |
| Methocel A4M | 0.30 | | | 0.11 | |
| Carboxy methyl cellulose | | 0.50 | | | |
| Water purified | 19.40 | 39.50 | 40.00 | 68.22 | 65.90 |
| **Total** | **100.00** | **100.00** | **100.00** | **100.00** | **100.00** |
| Propellant ( propane, isobutene and butane mixture | 8.00 | 8.00 | 8.00 | 8.00 | 8.00 |
| | | | | | |
| **Appearance:** | | | | | |
| foam quality | G-E | G-E | G-E | E | E |
| color | White | White | White | White | White |
| odor | No odor | No odor | No odor | No odor | No odor |
| Microscope | Crystals | Crystals | Crystals | Crystals | Crystals |

Good to excellent foam formulations were prepared with azelaic acid, surfactant, polymeric agent, and either another oil or propylene glycol water (and without any foam adjuvant). Reducing the levels of azelaic acid to lower levels eliminated the appearance of crystals (See below).

Surprisingly, it was possible to prepare good to excellent foam after removal of the polymeric agent. Thus, the presence of a polymeric agent, is surprisingly not essential for foam quality. Nonetheless, polymeric agents may still contribute to and can be significant with respect to foam and active agent stability.

IN SOLUTION

Example 4 - Azalaic acid composition

The following foamable vehicles were prepared and the quality of the resultant foam was ascertained.

|  | DCA022 |
|---|---|
| Azelaic acid | 6.00 |
| Propylene glycol | 50.00 |
| Polysorbate 80 | 20.00 |
| Xanthan gum | 0.30 |
| Methocel A4M | 0.30 |
| Water purified | 23.40 |
| **Total** | **100.00** |
| Propellant) propane, isobutene and butane mixture | 8.00 |
|  |  |
| **Appearance:** |  |
| foam quality | G-E |
| Color | White |
| Odor | No odor |
| Microscope | No crystals |

Lower azaleic acid levels provide a soluble composition. No solids or precipitates are observed. No crystals were observed at the level of microscopic examination. By no crystals means the ingredients dissolve and it is not a suspension.

**Claims**

1. A pharmaceutical or cosmetic composition comprising:

   a. a beneficially or therapeutically effective concentration of azelaic acid;
   b. a stabilizer selected from the group consisting of at least one surface-active agent; at least one polymeric agent; and mixtures thereof; and
   **characterised in that** the composition further comprises an organic solvent comprising capric/caprylic triglyceride and further comprises at least one polar carrier, selected from the group consisting of dimethyl isosorbide, glycerol, propylene glycol, hexylene glycol, terpene-ol, oleyl alcohol, lactic acid and glycolic acid;
   wherein the polymeric agent is about 0.01 % to about 5% by weight and is selected from the group consisting of a bioadhesive agent, a gelling agent, a film forming agent and a phase change agent;
   wherein the azelaic acid, stabilizer and solvent are selected to provide a composition that is substantially resistant to aging and to phase separation and or can substantially stabilize other active ingredients; and
   wherein if the composition is contained in a pressurized container and further comprises a liquefied hydrocarbon gas propellant at a concentration of about 3% to about 25% by weight of the total composition it is substantially flowable and provides a foam upon release.

2. The composition of Claim 1, wherein the composition is contained in a pressurized container and further comprises a liquefied hydrocarbon gas propellant at a concentration of about 3% to about 25% by weight of the total composition, is substantially flowable and provides a foam upon release and wherein the azelaic acid, stabilizer and solvent are selected to generate a breakable foam of good to excellent quality.

3. The composition of Claim 1, wherein the surface active agent is selected from the group consisting of a polysorbate, polyoxyethylene (20) sorbitan monostearate, polyoxyethylene (20) sorbitan monooleate, a polyoxyethylene fatty acid ester, Myrj 45, Myrj 49, Myrj 52, PEG-100 stearate, a polyoxyethylene alkyl ether, polyoxyethylene cetyl ether, brij 38, PEG-2 cetyl ether, brij 56, brij W1, a sucrose ester, a partial ester of sorbitol, sorbitan monolaurate, a monoglyceride, a diglyceride, isoceteth-20, steareth 2, glyceryl monostearate, steareth-21, PEG 40 stearate, sorbitan stearate, laureth 4, sorbitan monooleate, ceteareth 20, steareth 20, ceteth 20, macrogol cetostearyl ether, ceteth 2, PEG-30 dipolyhydroxystearate, sucrose distearate, cetomacrogol ether, cetearyl glucoside, polysorbate 20, methyl glucose sesquistearate, PEG 30 dipolyhydroxystearate, sucrose stearic acid esters, and mixtures thereof.

4. The composition of Claim 1, wherein the surface active agent is selected from the group consisting of a non-ethoxylated sorbitan ester, a glycerol fatty acid ester, a sucrose ester, an alkyl polyglycoside or is selected from the group consisting of sorbitan monopalmitate, sorbitan monostearate, sorbitan tristearate, sorbitan monooleate, sorbitan trioleate, sorbitan monolaurate, sorbitan sesquioleate, glycerol monostearate, glycerol monooleate, sucrose stearate, sucrose distearate, sucrose palmitate, sucrose laurate and lauryl diglucoside.

5. The composition of Claim 1 wherein the polymeric agent is selected from the group consisting of carbopol 934, pemulen TR2, xanthan gum, carboxy methyl cellulose, locust bean gum, sodium alginate, sodium caseinate, egg albumin, gelatin agar, carrageenin gum, quince seed extract, tragacanth gum, guar gum, cationic guars, hydroxypropyl guar gum, starch, an amine-bearing polymer, chitosan, alginic acid, hyaluronic acid, a chemically modified starch, a carboxyvinyl polymer, polyvinylpyrrolidone, polyvinyl alcohol, a polyacrylic acid polymer, a polymethacrylic acid polymer, polyvinyl acetate, a polyvinyl chloride polymer, a polyvinylidene chloride polymer, methylcellulose, hydroxypropyl cellulose, hydroxyethyl cellulose, hydroxy propylmethyl cellulose, methylhydroxyethylcellulose, methylhydroxypropylcellulose, hydroxyethylcarboxymethylcellulose, carboxymethyl cellulose, carboxymethylhydroxyethylcellulose, a cationic cellulose, aluminum starch octenylsuccinate (ASOS), PEG 1000, PEG 4000, PEG 6000 and PEG 8000.

6. The composition of Claim 1 wherein the at least one polar carrier is dimethyl isosorbide or propylene glycol and wherein the stabilizer is methylcellulose or xanthan gum.

7. The composition of Claim 2, further containing a foam adjuvant selected from the group consisting of a fatty alcohol having 15 or more carbons in their carbon chain; a fatty acid having 16 or more carbons in their carbon chain; fatty alcohols, derived from beeswax and including a mixture of alcohols, a majority of which has at least 20 carbon atoms in their carbon chain; a fatty alcohol having at least one double bond; a fatty acid having at least one double bond; a branched fatty alcohol; a branched fatty acid; and a fatty acid substituted with a hydroxyl group.

8. The composition of Claim 1, wherein the concentration of azelaic acid is between 5% and 25%.

9. The composition of Claim 1, wherein the pH of the composition is below 5.3.

10. The composition of Claim 1, further comprising an additional component selected from the group consisting of a modulating agent, a polar solvent, an anti perspirant, an anti-static agent, a buffering agent, a bulking agent, a chelating agent, a colorant, a conditioner, a deodorant, a diluent, a dye, an emollient, a fragrance, a humectant, an occlusive agent, a penetration enhancer, a perfuming agent, a permeation enhancer, a pH-adjusting agent, a preservative, a skin penetration enhancer, a sunscreen, a sun blocking agent, a sunless tanning agent, and a vitamin.

11. The composition of Claim 1, consisting of (% w/w)

| | |
|---|---|
| Azelaic Acid | 15.00 |
| Water | 51.90 |
| Caprylic/Capric triglyceride | 10.87 |
| Propylene glycol | 10.87 |
| Dimethyl isosorbide | 5.44 |
| PEG-40 stearate | 2.83 |
| Cetostearyl alcohol | 1.09 |

(continued)

| Polysorbate 80 | 0.98 |
|---|---|
| Glyceryl stearate | 0.54 |
| Xanthan qum | 0.27 |
| Methylcellulose A4M | 0.11 |
| Benzoic acid | 0.10 |
| NaOH (18% Solution) | to pH=4.5 |

**12.** Use of the composition according to any of Claims 1 to 11 for the preparation of a medicament for use in the treatment of dermatological disorders.

**13.** Use of the composition according to Claim 11 for the preparation of a medicament for use in the treatment of a skin disorder selected from acne, rosacea, a pigmentation disorder, a cell proliferation abnormality, a skin infection and a skin inflammation.

**14.** A composition according to any of Claims 1 to 11, for the treatment of a dermatological disorder.

**Patentansprüche**

**1.** Pharmazeutische oder kosmetische Zusammensetzung, die Folgendes beinhaltet:

a. eine nützliche oder therapeutisch wirksame Azelainsäurekonzentration;
b. einen Stabilisator, der aus der Gruppe bestehend aus wenigstens einem Tensid, wenigstens einem polymeren Mittel und Gemischen davon ausgewählt ist; und
**dadurch gekennzeichnet, dass** die Zusammensetzung ferner ein organisches Lösungsmittel, das Caprin/Capryl-Triglycerid beinhaltet, und ferner wenigstens einen polaren Träger beinhaltet, der aus der Gruppe bestehend aus Dimethylisosorbid, Glycerol, Propylenglykol, Hexylenglykol, Terpen-ol, Oleylalkohol, Milchsäure und Glykolsäure ausgewählt ist;
wobei das polymere Mittel etwa 0,01 bis etwa 5 Gew.-% ausmacht und aus der Gruppe bestehend aus einem bioadhäsiven Mittel, einem Geliermittel, einem Filmbildungsmittel und einem Phasenänderungsmittel ausgewählt ist;
wobei die Azelainsäure, der Stabilisator und das Lösungsmittel so gewählt werden, dass eine Zusammensetzung entsteht, die gegenüber Alterung und Phasenabscheidung im Wesentlichen resistent ist und/oder die andere Wirkstoffe im Wesentlichen stabilisieren kann; und
wobei die Zusammensetzung, wenn sie sich in einem Druckbehälter befindet und ferner ein Treibmittel aus verflüssigtem Kohlenwasserstoffgas in einer Konzentration von etwa 3 bis etwa 25 Gew.-% der Zusammensetzung enthält, im Wesentlichen fließfähig ist und bei Abgabe einen Schaumstoff bereitstellt.

**2.** Zusammensetzung nach Anspruch 1, wobei die Zusammensetzung in einem Druckbehälter enthalten ist und ferner ein Treibmittel aus verflüssigtem Kohlenwasserstoffgas in einer Konzentration von etwa 3 bis etwa 25 Gew.-% der Zusammensetzung enthält, im Wesentlichen fließfähig ist und bei Abgabe einen Schaumstoff bereitstellt, und wobei die Azelainsäure, der Stabilisator und das Lösungsmittel zum Erzeugen eines zerbrechlichen Schaumstoffs von guter bis ausgezeichneter Qualität ausgewählt werden.

**3.** Zusammensetzung nach Anspruch 1, wobei das Tensid ausgewählt ist aus der Gruppe bestehend aus Polysorbat, Polyoxyethylen (20) Sorbitanmonostearat, Polyoxyethylen (20) Sorbitanmonooleat, einem Polyoxyethylen-Fettsäureester, Myrj 45, Myrj 49, Myrj 52, PEG-100 Stearat, einem Polyoxyethylenalkylether, Polyoxyethylencetylether, brij 38, PEG-2-Cetylether, brij 56, brij W1, einem Saccharoseester, einem Sorbitolpartialester, Sorbitanmonolaurat, einem Monoglycerid, einem Diglycerid, Isoceteth-20, Steareth 2, Glycerylmonostearat, Steareth-21, PEG-40 Stearat, Sorbitanstearat, Laureth 4, Sorbitanmonooleat, Ceteareth 20, Steareth 20, Ceteth 20, Macrogolcetostearylether, Ceteth 2, PEG-30 Dipolyhydroxystearat, Saccharosedistearat, Cetomacrogolether, Cetearylglucosid, Polysorbat 20, Methylglucosesesquistearat, PEG-30 Dipolyhydroxystearat, Saccharosestearinestersäure und Gemischen davon.

4. Zusammensetzung nach Anspruch 1, wobei das Tensid ausgewählt ist aus der Gruppe bestehend aus einem nichtethoxylierten Sorbitanester, einem Glycerolfettsäureester, einem Saccharoseester, einem Alkylpolyglykosid, oder ausgewählt ist aus der Gruppe bestehend aus Sorbitanmonopalmitat, Sorbitanmonostearat, Sorbitantristearat, Sorbitanmonooleat, Sorbitantrioleat, Sorbitanmonolaurat, Sorbitansesquioleat, Glycerolmonostearat, Glycerolmonooleat, Saccharosestearat, Saccharosedistearat, Saccharosepalmitat, Saccharoselaurat und Lauryldiglukosid.

5. Zusammensetzung nach Anspruch 1, wobei das polymere Mittel ausgewählt ist aus der Gruppe bestehend aus Carbopol 934, Pemulen TR2, Xanthangummi, Carboxymethylcellulose, Johannisbrotkernmehl, Natriumalginat, Natriumcaseinat, Eieralbumin, Gelatine-Agar, Carrageeningummi, Quittensamenextrakt, Tragantgummi, Guargummi, kationischen Guars, Hydroxypropylguargummi, Stärke, einem amintragenden Polymer, Chitosan, Algininsäure, Hyaluronsäure, einer chemisch modifizierten Stärke, einem Carboxyvinylpolymer, Polyvinylpyrrolidon, Polyvinylalkohol, einem Polyacrylsäurepolymer, einem Polymethacrylsäurepolymer, Polyvinylacetat, einem Polyvinylchloridpolymer, einem Polyvinylidenchloridpolymer, Methylcellulose, Hydroxypropylcellulose, Hydroxyethylcellulose, Hydroxypropylmethylcellulose, Methylhydroxyethylcellulose, Methylhydroxypropylcellulose, Hydroxyethylcarboxymethylcellulose, Carboxymethylcellulose, Carboxymethylhydroxyethylcellulose, einer kationischen Cellulose, Aluminiumstärkeoctenylsuccinat (ASOS), PEG 1000, PEG 4000, PEG 6000 und PEG 8000.

6. Zusammensetzung nach Anspruch 1, wobei der wenigstens eine polare Träger Dimethylisosorbid oder Propylenglykol ist und wobei der Stabilisator Methylcellulose oder Xanthangummi ist.

7. Zusammensetzung nach Anspruch 2, die ferner ein Schaumstoffadjuvans enthält, das ausgewählt ist aus der Gruppe bestehend aus einem Fettalkohol mit 15 oder mehr Kohlenstoffatomen in der Kohlenstoffkette; einer Fettsäure mit 16 oder mehr Kohlenstoffen in der Kohlenstoffkette; Fettalkoholen, abgeleitet von Bienenwachs und mit einem Gemisch von Alkoholen, von denen eine Mehrzahl wenigstens 20 Kohlenstoffatome in der Kohlenstoffkette haben; einem Fettalkohol mit wenigstens einer Doppelbindung; einer Fettsäure mit wenigstens einer Doppelbindung; einem verzweigten Fettalkohol; einer verzweigten Fettsäure; und einer durch eine Hydroxylgruppe substituierten Fettsäure.

8. Zusammensetzung nach Anspruch 1, wobei die Azelainsäurekonzentration zwischen 5 % und 25 % liegt.

9. Zusammensetzung nach Anspruch 1, wobei der pH-Wert der Zusammensetzung unter 5,3 liegt.

10. Zusammensetzung nach Anspruch 1, die ferner eine zusätzliche Komponente beinhaltet, die ausgewählt ist aus der Gruppe bestehend aus einem Modulierungsmittel, einem polaren Lösungsmittel, einem Antitranspirant, einem antistatischen Mittel, einem Puffermittel, einem Füllstoff, einem Chelatierungsmittel, einem Farbstoff, einem Konditionierer, einem Deodorant, einem Verdünnungsmittel, einem Farbstoff, einem Weichmacher, einem Duftstoff, einem Befeuchtungsmittel, einem Okklusionsmittel, einem Penetrationsverbesserer, einem Parfümiermittel, einem Permeationsverbesserer, einem pH-Einstellmittel, einem Konservierungsmittel, einem Hautpenetrationsverbesserer, einem Sonnenschutzmittel, einem Sonnenblockiermittel, einem sonnenlosen Bräunungsmittel und einem Vitamin.

11. Zusammensetzung nach Anspruch 1, bestehend aus (Gew.-%):

| | |
|---|---|
| Azelainsäure | 15,00 |
| Wasser | 51,90 |
| Caprin/Capryl-Triglycerid | 10,87 |
| Propylenglykol | 10,87 |
| Dimethylisosorbid | 5,44 |
| PEG-40 Stearat | 2,83 |
| Cetostearylalkohol | 1,09 |
| Polysorbat 80 | 0,98 |
| Glycerylstearat | 0,54 |
| Xanthangummi | 0,27 |
| Methylcellulose A4M | 0,11 |

(fortgesetzt)

| Benzoesäure | 0,10 |
|---|---|
| NaOH (18%ige Lösung) | bis pH=4,5 |

**12.** Verwendung der Zusammensetzung nach einem der Ansprüche 1 bis 11 für die Herstellung eines Medikaments zur Verwendung bei der Behandlung von dermatologischen Störungen.

**13.** Verwendung der Zusammensetzung nach Anspruch 11 für die Herstellung eines Medikaments zur Verwendung bei der Behandlung einer Hautstörung, die aus Akne, Rosacea, einer Pigmentierungsstörung, einer Zellproliferationsanomalität, einer Hautinfektion und einer Hautentzündung ausgewählt ist.

**14.** Zusammensetzung nach einem der Ansprüche 1 bis 11 zur Behandlung einer dermatologischen Störung.

**Revendications**

**1.** Composition pharmaceutique ou cosmétique comprenant :

a. une concentration efficace en termes bénéfiques ou thérapeutiques d'acide azélaïque ;
b. un stabilisant sélectionné à partir du groupe constitué d'au moins un agent tensioactif ; d'au moins un agent polymère ; et leurs mélanges ; et
**caractérisée en ce que** la composition comprend en outre un solvant organique comprenant un triglycéride caprique/caprylique et comprend en outre au moins un support polaire, sélectionné à partir du groupe constitué d'isosorbide de diméthyle, de glycérol, de propylène glycol, d'hexylène glycol, de terpénol, d'alcool oléylique, d'acide lactique et d'acide glycolique ;
où l'agent polymère représente environ 0,01 % à environ 5 % en poids et est sélectionné à partir du groupe constitué d'un agent bioadhésif, d'un agent gélifiant, d'un agent filmogène et d'un agent de changement de phase ;
où l'acide azélaïque, le stabilisant et le solvant sont sélectionnés afin de fournir une composition qui est substantiellement résistante au vieillissement et à la séparation de phases et/ou peut substantiellement stabiliser d'autres principes actifs ; et
où si la composition est contenue dans un contenant sous pression et comprend en outre un gaz propulseur hydrocarboné liquéfié à une concentration d'environ 3 % à environ 25 % en poids de la composition totale, elle est substantiellement liquide et fournit une mousse lors de la libération.

**2.** Composition selon la revendication 1, où la composition est contenue dans un contenant sous pression et comprend en outre un gaz propulseur hydrocarboné liquéfié à une concentration d'environ 3 % à environ 25 % en poids de la composition totale, est substantiellement liquide et fournit une mousse lors de la libération, et où l'acide azélaïque, le stabilisant et le solvant sont sélectionnés afin de générer une mousse cassante de bonne ou d'excellente qualité.

**3.** Composition selon la revendication 1, où l'agent tensioactif est sélectionné à partir du groupe constitué d'un polysorbate, de monostéarate polyoxyéthylénique (20) de sorbitanne, de mono-oléate polyoxyéthylénique (20) de sorbitanne, d'un ester d'acide gras polyoxyéthylénique, de Myrj 45, de Myrj 49, de Myrj 52, de stéarate de PEG-100, d'un éther d'alkyle polyoxyéthylénique, d'éther cétylique polyoxyéthylénique, de brij 38, d'éther cétylique de PEG-2, de brij 56, de brij W1, d'un ester de saccharose, d'un ester partiel de sorbitol, de monolaurate de sorbitanne, d'un monoglycéride, d'un diglycéride, d'isoceteth-20, de stéareth 2, de monostéarate de glycéryle, de stéareth-21, de stéarate de PEG 40, de stéarate de sorbitanne, de laureth 4, de mono-oléate de sorbitanne, de céteareth 20, de stéareth 20, de céteth 20, d'éther cétostéarylique de macrogol, de céteth 2, de dipolyhydroxystéarate de PEG 30, de distéarate de saccharose, d'éther de cétomacrogol, de glucoside de cétéaryle, de polysorbate 20, de sesquistéarate de méthyl glucose, de dipolyhydroxystéarate de PEG 30, d'esters d'acide stéarique de saccharose, et leurs mélanges.

**4.** Composition selon la revendication 1, où l'agent tensioactif est sélectionné à partir du groupe constitué d'un ester non-éthoxylé de sorbitanne, d'un ester d'acide gras de glycérol, d'un ester de saccharose, d'un polyglycoside d'alkyle ou est sélectionné à partir du groupe constitué de monopalmitate de sorbitanne, de monostéarate de sorbitanne, de tristéarate de sorbitanne, de mono-oléate de sorbitanne, de trioléate de sorbitanne, de monolaurate de sorbitanne, de sesquioléate de sorbitanne, de monostéarate de glycérol, de mono-oléate de glycérol, de stéarate de saccharose, de distéarate de saccharose, de palmitate de saccharose, de laurate de saccharose et de diglucoside de lauryl.

5. Composition selon la revendication 1, où l'agent polymère est sélectionné à partir du groupe constitué de carbopol 934, de pemulen TR2, de gomme xanthane, de carboxyméthylcellulose, de gomme de caroube, d'alginate de sodium, de caséinate de sodium, d'albumine d'oeuf, de gélose à la gélatine, de gomme de carraghénine, d'extrait de graine de coing, de gomme adragante, de gomme de guar, de guars cationiques, de gomme de guar d'hydroxypropyle, d'amidon, d'un polymère porteur d'amine, de chitosane, d'acide alginique, d'acide hyaluronique, d'un amidon chimiquement modifié, d'un polymère carboxyvinylique, de polyvinylpyrrolidone, d'alcool polyvinylique, d'un polymère d'acide polyacrylique, d'un polymère d'acide polyméthacrylique, d'acétate de polyvinyle, d'un polymère de chlorure polyvinylique, d'un polymère de chlorure de polyvinylidène, de méthylcellulose, d'hydroxypropylcellulose, d'hydroxyéthylcellulose, d'hydroxypropylméthylcellulose, de méthylhydroxyéthylcellulose, de méthylhydroxypropylcellulose, d'hydroxyéthylcarboxyméthylcellulose, de carboxyméthylcellulose, de carboxyméthylhydroxyéthylcellulose, d'une cellulose cationique, d'octénylsuccinate d'amidon aluminium (ASOS), de PEG 1000, de PEG 4000, de PEG 6000 et de PEG 8000.

6. Composition selon la revendication 1, où l'au moins un support polaire est de l'isosorbide de diméthyle ou du propylèneglycol et où le stabilisant est de la méthylcellulose ou de la gomme xanthane.

7. Composition selon la revendication 2, contenant en outre un adjuvant de mousse sélectionné à partir du groupe constitué d'un alcool gras ayant 15 carbones ou plus dans sa chaîne carbonée ; d'un acide gras ayant 16 carbones ou plus dans sa chaîne carbonée ; d'alcools gras, dérivés de la cire d'abeille et incluant un mélange d'alcools, dont une majorité ont au moins 20 atomes de carbone dans leur chaîne carbonée ; d'un alcool gras ayant au moins une double liaison ; d'un acide gras ayant au moins une double liaison ; d'un alcool gras ramifié ; d'un acide gras ramifié ; et d'un acide gras substitué par un groupe hydroxyle.

8. Composition selon la revendication 1, où la concentration d'acide azélaïque est comprise entre 5 % et 25 %.

9. Composition selon la revendication 1, où le pH de la composition est inférieur à 5,3.

10. Composition selon la revendication 1, comprenant en outre un composant supplémentaire sélectionné à partir du groupe constitué d'un agent de modulation, d'un solvant polaire, d'un anti-transpirant, d'un agent antistatique, d'un agent tampon, d'un agent gonflant, d'un agent chélatant, d'un colorant, d'un conditionneur, d'un déodorant, d'un diluant, d'une matière colorante, d'un émollient, d'un parfum, d'un humectant, d'un agent occlusif, d'un activateur de pénétration, d'un agent parfumant, d'un agent augmentant la pénétration, d'un agent d'ajustement du pH, d'un conservateur, d'un activateur de pénétration cutanée, d'un écran solaire, d'un agent de blocage solaire, d'un agent de bronzage sans soleil, et d'une vitamine.

11. Composition selon la revendication 1, constituée des composants suivants (% M/M) :

| | |
|---|---|
| Acide azélaïque | 15,00 |
| Eau | 51,90 |
| Triglycéride caprylique/caprique | 10,87 |
| Propylèneglycol | 10,87 |
| Isosorbide de diméthyle | 5,44 |
| Stéarate de PEG-40 | 2,83 |
| Alcool cétostéarylique | 1,09 |
| Polysorbate 80 | 0,98 |
| Stéarate de glycéryle | 0,54 |
| Gomme xanthane | 0,27 |
| Méthylcellulose A4M | 0.11 |

(suite)

| Acide benzoïque | 0,10 |
|---|---|
| NaOH (solution à 18 %) | vers un pH = 4,5 |

12. Utilisation de la composition selon l'une quelconque des revendications 1 à 11 pour la préparation d'un médicament destiné à être utilisé dans le traitement de troubles dermatologiques.

13. Utilisation de la composition selon la revendication 11 pour la préparation d'un médicament destiné à être utilisé dans le traitement d'une maladie de la peau sélectionnée parmi l'acné, la rosacée, un trouble de la pigmentation, une anomalie de la prolifération cellulaire, une infection de la peau et une inflammation de la peau.

14. Composition selon l'une quelconque des revendications 1 à 11 pour le traitement d'un trouble dermatologique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6180669 B
- US 20040191196 A
- US 20060233721 A
- US 050232869 B
- US 050205086 B
- US 060018937 B
- US 050271596 B
- US 060269485 B
- US 070020304 B
- US 060193789 B
- US 73254707 A
- US 78918606 P
- US 0815948 A
- US 81863406 P
- US 84314006 P
- WO 2004037225 A

**Non-patent literature cited in the description**

- **HONG-RONG WANG ; KENG-MING CHEN.** *Colloids and Surfaces A: Physicochemical and Engineering Aspects,* 15 June 2006, vol. 281 (1-3), 190-193
- **VAN SLYKE.** *J. Biol. Chem.,* 1922, vol. 52, 525
- Handbook of Pharmaceutical Additives. Gower